# EUROPÄISCHE PATENTSCHRIFT

(11) **EP 3 705 080 B1**
(45) Veröffentlichungstag und Bekanntmachung des Hinweises auf die Patenterteilung: **26.05.2021**
(21) Anmeldenummer: 19161770.3
(22) Anmeldetag: 08.03.2019
(51) Int. Cl.: A61C 13/00, G06F 17/00, G06F 30/00, G16H 20/40

(54) **COMPUTERIMPLEMENTIERTES MODELLIEREN EINES PATIENTENINDIVIDUELLEN ZAHNERSATZTEILS**
COMPUTER IMPLEMENTED MODELLING OF A PATIENT-SPECIFIC DENTAL RESTORATION
MODÉLISATION INFORMATIQUE D'UNE PROTHÈSE DENTAIRE SPÉCIFIQUE AU PATIENT

(43) Veröffentlichungstag der Anmeldung: 09.09.2020
(73) Patentinhaber: Exocad GmbH, 64293 Darmstadt (DE)
(72) Erfinder: CHIOSA, Iurie, 64331 Weiterstadt (DE); GERTH, Maik, 64283 Darmstadt (DE); STEINBRECHER, Tillmann, 64342 Seeheim-Jugenheim (DE)
(74) Vertreter: Richardt Patentanwälte PartG mbB

(56) Entgegenhaltungen:
- EP-A1- 2 134 290
- DE-A1-102011 005 899
- US-A1- 2013 282 351
- US-A1- 2018 085 203

## Beschreibung

Die Erfindung betrifft ein computerimplementiertes Verfahren zum Modellieren eines patientenindividuellen Zahnersatzteils, ein Computerprogrammprodukt sowie ein Computersystem und ein Bearbeitungssystem zum Ausführen des Verfahrens. Zahnersatzteile werden üblicherweise maschinell oder vollständig in Handarbeit gefertigt. Da neben einer präzisen Bemaßung des Zahnersatzteils dessen ästhetisches Erscheinungsbild insbesondere im Wechselspiel mit den Gegebenheiten eines Patientengebisses, in welchem das entsprechende Zahnersatzteil anzuordnen ist, eine wichtige Rolle spielt, erfolgt selbst im Falle einer maschinellen Herstellung üblicherweise eine Nachbearbeitung von Hand. Da im Allgemeinen keine zwei Patientengebisse identisch sind, ergeben sich hohe Anforderungen an ein Modellieren eines patientenindividuellen Zahnersatzteils, welches sich sowohl von seinen Maßen als auch von seinem Erscheinungsbild präzise anpassen sollte. Daher erfolgt selbst im Falle einer maschinellen Herstellung des Zahnersatzteils üblicherweise vorab eine Modellierung des entsprechenden Zahnersatzteils von Hand unter Verwendung von Computermodellen. Dabei erweisen sich bekannte Modellierungsverfahren jedoch als aufwendig und unhandlich. So muss jede einzelne Veränderung des Modells individuell von Hand vorgenommen werden. Hierbei kann es insbesondere wiederholt dazu kommen, dass eine Veränderung des Zahnmodells dazu führt, das zuvor bereits angepasste Teilbereiche des Modells infolge der zusätzlichen Veränderung nicht mehr passen und ebenfalls angeglichen werden müssen.

Die DE 10 2011 005899 A1 beschreibt ein Verfahren zur Bearbeitung eines bei der Planung eines Zahnersatzes erzeugten ersten virtuellen dreidimensionalen Zahnmodells eines Zahnersatzteils mittels eines virtuellen Werkzeugs. Das erste virtuelle Zahnmodell wird mittels des virtuellen Werkzeugs bearbeiten, wobei während der Bearbeitung mittels des virtuellen Werkzeugs das erste virtuelle Zahnmodell mittels eines Computers automatisch so angepasst wird, dass bestimmte bei der Planung des Zahnersatzes festgelegte Randbedingungen erfüllt bleiben.

Die US 2018/085203 A1 beschreibt ein computerimplementiertes Verfahren zum Entwerfen einer Zahnrestauration auf einem Display, wobei das Verfahren ein Bereitstellen einer virtuellen dreidimensionalen Darstellung von mindestens einem Teil der Zahnsituation des Patienten umfasst. Das Verfahren umfasst das Anzeigen eines virtuellen dreidimensionalen Zahnrestaurationsmodells in einer Ausrichtung mit der virtuellen dreidimensionalen Darstellung. Das Verfahren umfasst auch das Bereitstellen eines Entwurfswerkzeugs, das auswählbar ist, um mindestens einen Teil des dreidimensionalen Zahnrestaurationsmodells zu verformen. Wenn dieses Design-Tool ausgewählt ist, kann eine Linie auf einer Oberfläche des dreidimensionalen Zahnrestaurationsmodells gezeichnet werden.

Die US 2013/282351 A1 beschreibt ein Verfahren zur Bestimmung virtueller Zahnrestaurationen auf Basis von Scandaten oraler Strukturen, wobei eine Modelldatenbank verwendet wird, die eine Anzahl parametrisierter Zahnmodelle für jeden von mehreren Zahntypen umfasst, wobei die Parametrisierung auf Grundlage von Modellparametern ausgeführt wird, welche Positionsparameter und/oder Formparameter umfassen, und wobei jedes Zahnmodell mit einer Anzahl von Zahnmodellendes gleichen Zahntyps verknüpft ist, und wobei für jeden gewünschten Zahntyp ein optimales Zahnmodell in der Modelldatenbank unter Verwendung eines iterativen Verfahrens ermittelt wird, bei welchem zunächst mindestens ein Startzahnmodell des gewünschten Zahntyps aus der Modelldatenbank ausgewählt wird, und anschließend ausgehend von diesem Startzahnmodell in jedem Iterationsschritt ein Zahnmodell auf einen Qualitätswert hin überprüft wird, wobei zum Zweck einer Individualisierung das aktuell getestete Zahnmodell jeweils angepasst wird an die Scan-Daten durch Variation von Modellparametern und wobei für diese Individualisierung ein Qualitätswert berechnet wird.

Die EP 2 134 290 A1 beschreibt ein Verfahren zur automatischen oder halbautomatischen Planung einer Zahnbehandlung für einen Patienten, welches umfasst: (a) Erhalten von Daten über einen zu behandelnden Bereich und Daten über ein Gesicht eines Patienten; (b) Durchführen einer computergestützten Analyse der Daten, um Eigenschaften von mindestens dem Gesicht des Patienten zu bestimmen; (c) Erzeugen eines modifizierten Zahnkonfiguration unter Verwendung eines Satzes gespeicherter Regeln, welche die bestimmten Gesichtseigenschaften verwenden. Eine dreidimensionale Darstellung simuliert das Erscheinungsbild der modifizierten Zahnkonfiguration und das Gesicht des Patienten, das den Behandlungsbereich umgibt.

Der Erfindung liegt die Aufgabe zugrunde, ein verbessertes Verfahren zum Modellieren von Zahnersatzteilen zu ermöglichen.

Die der Erfindung zugrundeliegende Aufgabe wird jeweils mit den Merkmalen der unabhängigen Patentansprüche gelöst. Ausführungsformen der Erfindung sind in den abhängigen Patentansprüchen angegeben.

Die Erfindung umfasst ein computerimplementiertes Verfahren zum Modellieren eines patientenindividuellen Zahnersatzteils, wobei das Verfahren umfasst:
- Bereitstellen eines digitalen dreidimensionalen Patientensituationsmodells, wobei das Patientensituationsmodell patientenspezifische Begrenzungsoberflächen von ein oder mehreren Objekten eines Patientengebisses definiert, welche eine Patientensituationsgeometrie definieren, an welche das Zahnersatzteil im Zuge der Modellierung anzupassen ist,
- Bereitstellen eines ersten digitalen dreidimensionalen Zahnersatzteilmodells in einem Ausgangszustand, wobei das erste Zahnersatzteilmodell zahnersatzteilspezifische Begrenzungsoberflächen des Zahnersatzteils definiert, welche eine Zahnersatzteilgeometrie definieren,
   wobei das erste Zahnersatzteilmodell in dem Ausgangszustand eine Zahnersatzteilgeometrie in Form einer Ausgangsgeometrie aufweist,
- Bereitstellen ein oder mehrere unter Verwendung der patientenspezifischen Begrenzungsoberflächen definierten geometrischen Anpassungskriterien, welches durch die zahnersatzteilspezifische Begrenzungsoberflächen im Zuge eines patientenindividuellen Anpassens der Zahnersatzteilgeometrie an die Patientensituationsgeometrie einzuhalten sind,
- patientenindividuelles Anpassen der Zahnersatzteilgeometrie des ersten Zahnersatzteilmodells an die Patientensituationsgeometrie des Patientensituationsmodells,
   wobei das patientenindividuelle Anpassen ein Anordnen des ersten Zahnersatzteilmodells in einer von dem Patientensituationsmodell für das Zahnersatzteil bereitgestellten Ausgangsposition umfasst,
   wobei das patientenindividuelle Anpassen ferner ein wiederholtes interaktives Ausführen benutzerdefinierter Veränderungen an dem angeordneten ersten Zahnersatzteilmodell umfasst, wobei das ersten Zahnersatzteilmodell im Zuge jeder der benutzerdefinierten Veränderungen jeweils dynamisch eine Sequenz von Zwischenzuständen durchläuft bis ein aus der jeweiligen benutzerdefinierten Veränderung resultierender Änderungszustand erreicht wird, wobei für jeden der entsprechenden Zwischenzustände sowie den resultierenden Änderungszustand jeweils automatisch aus der Ausgangsgeometrie des ersten Zahnersatzteilmodells eine zustandsspezifische Zustandsgeometrie des ersten Zahnersatzteilmodells unter Einhaltung der geometrischen Anpassungskriterien berechnet wird,
   wobei jede der benutzerdefinierten Veränderungen mittels einer grafischen Benutzeroberfläche auf einer Anzeigevorrichtung angezeigt wird, wobei das Anzeigen der jeweiligen benutzerdefinierten Veränderung jeweils ein Anzeigen des dynamischen Durchlaufens der jeweiligen Sequenz von Zwischenzuständen bis zum Erreichen des jeweiligen Änderungszustands durch das erste Zahnersatzteilmodell mit den jeweiligen hierfür berechneten zustandsspezifische Zustandsgeometrien umfasst,
- Verwenden einer aus dem patientenindividuellen Anpassen des ersten Zahnersatzteilmodells resultierenden Änderungsgeometrie zum Bereitstellen einer patientenindividuellen Zahnersatzteilgeometrie für das Herstellen des patientenindividuellen Zahnersatzteils.

Ausführungsmodelle können den Vorteil haben, dass sie es einem Nutzer ermöglichen, ein digitales dreidimensionales Zahnersatzteilmodell ausgehend von einem Ausgangszustand an die individuellen Gegebenheiten einer Patientensituationsgeometrie anzupassen. Eine Patientensituationsgeometrie ist definiert von ein oder mehreren in einem Patientengebiss angeordneten Objekten, an welche das Zahnersatzteilmodell anzupassen ist. Die vorgegebenen geometrischen Anpassungskriterien stellen sicher, dass jede benutzerdefinierte Veränderung unter Einhaltung der entsprechenden Anpassungskriterien erfolgt. Mit anderen Worten kann somit verhindert werden, dass durch eine benutzerdefinierte Veränderung des Zahnersatzteilmodells ein Zustand eingestellt wird, welcher ein geometrisches Anpassungskriterium verletzt. Ausführungsformen haben den Vorteil, dass dadurch verhindert werden kann, dass infolge benutzerdefinierter Veränderungen Nachbesserungen an dem Zahnersatzteilmodell notwendig werden, um die entsprechenden Anpassungskriterien wieder zu erfüllen. Somit kann die Problematik vermieden werden, dass eine benutzerdefinierte Veränderung dazu führt, dass eine Nachbesserung notwendig wird, um ein durch zuvor vorgenommene Veränderungen bereits erfülltes Anpassungskriterium, das durch den neuen Zustand verletzt wird, wieder zu erfüllen. Ein entsprechendes Nachbessern kann dazu führen, dass die vorherige benutzerdefinierte Veränderung zumindest soweit rückgängig gemacht wird, dass diese teilweise wiederholt werden muss, was wiederum zu Problemen mit dem Erfüllen des entsprechenden Anpassungskriteriums führen kann. Durch ein festes Vorgeben der entsprechenden geometrischen Anpassungskriterien kann sichergestellt werden, dass ein aufwendiges iteratives Annähern an ein simultanes Erfüllen der entsprechenden Anpassungskriterien einerseits und benutzerdefinierter Veränderungen zum patientenindividuellen Anpassen des Zahnersatzteilmodells andererseits vermieden werden kann.

Ferner erlaubt ein Anzeigen eines dynamischen Durchlaufs einer Sequenz von Zwischenzuständen, dass ein Nutzer ein besseres Verständnis für die Außenwirkungen der von ihm vorgenommenen benutzerdefinierten Veränderungen erhält. Insbesondere kann der Nutzer so erkennen, ob einer der dynamisch durchlaufenden Zwischenzuständen einen von ihm erstrebten vorteilhaften Änderungszustand des Zahnersatzteilmodells darstellt. Ist dies der Fall, so kann der Nutzer durch Rückgängigmachen eines Teils der benutzerdefinierten Veränderungen zu dem entsprechenden Zwischenzustand gelangen. Dadurch wird die Handhabbarkeit des Verfahrens erhöht und die Bearbeitungszeit kann deutlich reduziert werden.

Hierbei ist insbesondere von Vorteil, dass sowohl für die Zwischenzustände als auch für die resultierenden Änderungszustände die zustandsspezifische Zustandsgeometrie jeweils automatisch aus der Ausgangsgeometrie berechnet wird. Mit anderen Worten erfolgt eine Berechnung der Zustandsgeometrien der Zwischenzustände und/oder des Änderungszustands jeweils für sich aus der Ausgangsgeometrie. Ein Berechnen der einzelnen Zustandsgeometrien jeweils aus der Ausgangsgeometrie kann sicherstellen, dass jede durch den Nutzer vorgenommene benutzerdefinierte Veränderung problemlos zurückgenommen werden kann. Hierdurch kann insbesondere ein Anpassen des Zahnersatzteilmodells an einen zuvor durchlaufenen Zwischenzustand erleichtert werden. Insbesondere kann so eine Situation vermieden werden, in der sich der Nutzer gezwungen sieht, mit der Modellierung von Neuem zu beginnen, da eine von ihm vorgenommene Kombination aus benutzerdefinierten Veränderungen nicht mehr zurückgenommen werden kann. Diese Situation kann insbesondere dann entstehen, wenn jeweils von Zustand zu Zustand eine Anpassung des Zahnersatzteilmodells unter Verwendung numerischer Näherungsverfahren berechnet wird. So kann es beispielsweise infolge numerischer Effekte, wie etwa Rundungsfehler, dazu kommen, dass ohne einen festen Bezugszustand des Zahnersatzteilmodells Veränderungen grundsätzlich nicht vollständig rückgängig zu machen sind. Im vorliegenden Fall wird ein fester Bezugszustand in Form der Ausgangsgeometrie bereitgestellt. Ausführungsformen können somit ein wesentlich einfacher zu handhabendes und schnelleres Modellieren patientenindividueller Zahnersatzteile ermöglichen.

Ausführungsformen können zudem den Vorteil haben, da die Berechnung der Änderungszustände jeweils individuell ausgehend von der Ausgangsgeometrie erfolgt, dass für jeden Änderungszustands ein Satz von Änderungsdaten erstellt werden kann, welcher die für den entsprechenden der Änderungszustand vorgenommen Änderungen gegenüber der Ausgangsgeometrie definiert. Ein solcher Satz Änderungsdaten ermöglicht es für ein erstes Zahnersatzteilmodell vorgenommene Änderungen in einfacher Form auf ein zweites Zahnersatzteilmodell zu übertragen. Beispielsweise werden hierzu die in dem Satz Änderungsdaten definierten Änderungen auf das zweite Zahnersatzteilmodell angewendet. Da es sich um Änderungen handelt, welche direkt aus der Ausgangsgeometrie berechnet wurden, können numerische und/oder zahnersatzteilmodellspezifische Ungenauigkeiten bei der Übertragung reduziert werden.

Die zuvor beschriebenen Vorteile kommen insbesondere zum Tragen, wenn eine Mehrzahl von Zahnersatzteilen, insbesondere Zahnersatzteile, welche voneinander abhängig sind, zu modellieren sind.

Unter einem Zahnersatzteil wird jegliche Form von Objekt zum Anordnen in oder an einem Patientengebiss verstanden, welches zum Ersetzen und/oder zum Ergänzen eines oder mehrerer fehlender oder vorhandener natürlicher Zähne und/oder eines oder mehrerer Teile davon. Zahnersatzteile können beispielsweise Brücken, Inlays, Overlays, Kronen oder Ähnliches umfassen. Zahnersatzteile werden aus einem Zahnersatzmetrial hergestellt, z.B. aus Keramiken, wie Zinkoxidkeramik, Aluminiumoxidkeramik, aus Metallen oder Metalllegierungen hergestellt. Beispielsweise werden CrCo-Legierungen, Gold oder Goldlegierungen verwendet. Ferner kann Zahnersatzmetrial auch Kunststoff umfassen.

Eine Zahnersatzteilgeometrie bezeichnet eine durch zahnersatzteilspezifischen Begrenzungsoberflächen gebildete geometrische Form und/oder Formen eines Zahnersatzteilmodells. Diese Formen lassen sich quantitativ beschreiben durch geometrische Größen oder Verhältnisse von geometrischen Größen, wie z.B. Abstände, Längen, Breiten, Höhen, Winkel, Punkte, (Ober-) Flächen oder Volumen. Ein Zahnersatzteilmodell kann aus einer Sammlung vordefinierter Zahnersatzteilmodelle, d.h. einer Bibliothek, ausgewählt oder für einen konkreten Anwendungsfall erzeugt werden. Hierzu können physikalische Objekte eines Patientengebisses als Vorlage für ein digitales dreidimensionales Modell dienen oder bereits bestehende patientenspezifische digitale dreidimensionales Modelle als Vorlage und/oder Ausgangsmodell verwendet werden.

Eine Patientensituationsgeometrie bezeichnet eine durch patientenspezifischen Begrenzungsoberflächen von ein oder mehrere vorhandenen und/oder zu ergänzenden Objekten eines Patentengebisses gebildete geometrische Form und/oder Formen eines Patientensituationsmodells bzw. der von dem Patientensituationsmodell umfassten Modellen der entsprechenden Objekte des Patentengebisses. Diese Formen lassen sich quantitativ beschreiben durch geometrische Größen oder Verhältnisse von geometrischen Größen, wie z.B. Abstände, Längen, Breiten, Höhen, Winkel, Punkte, (Ober-) Flächen oder Volumen. Ein Patientensituationsmodell gibt einen Gebisszustand und/oder einen Zustand eines oder mehrerer Teile eines Patientengebisses wieder, in welchen das Zahnersatzteil einzupassen bzw. an welches es anzupassen ist. Dabei beruht das Patientensituationsmodell beispielsweise auf einer Vermessung eines Patientengebisses und/oder ein oder mehreren Teilen eines Patientengebisses.

Nach Ausführungsformen erfolgt die Anzeige der benutzerdefinierten Veränderungen jeweils simultan zu ihrer Eingabe. Ausführungsformen können den Vorteil haben, dass der Nutzer so unmittelbar eine Visualisierung der von ihm vorgenommenen Anpassungen erhält. Die Berechnung der Zwischenzustände sowie des resultierenden Änderungszustands erfolgt dabei on-the-fly in Echtzeit.

Nach Ausführungsformen erfolgt das Anpassen der Zahnersatzteilgeometrie unter Beibehaltung morphologischer Merkmale, welche von der Ausgangsgeometrie definiert und für das Zahnersatzteil charakteristisch sind. Ausführungsformen können den Vorteil haben, dass das entsprechende Zahnersatzteil stets seinen funktionalen und/oder ästhetischen Grundcharakter beibehält.

Nach Ausführungsformen umfasst das Bereitstellen der resultierenden Änderungsgeometrie ein Verwenden der resultierenden Änderungsgeometrie als patientenindividuelle Zahnersatzteilgeometrie. Ausführungsformen können den Vorteil haben, dass die resultierende Änderungsgeometrie beispielsweise indirekt als patientenindividuelle Zahngeometrie verwendet werden kann, beispielsweise zum Herstellen des Zahnersatzteils mittels eines geeigneten Bearbeitungsverfahrens, wie etwa einem CAM-Verfahren oder einem Rapid-Prototyping-Verfahren. Hierbei kann es sich um ein spanabhebendes Bearbeitungsverfahren, z. B. CNC-Fräsen, oder ein additives Bearbeitungsverfahren, z.B. 3D-Drucken, handeln.

Nach Ausführungsformen umfasst das Bereitstellen der resultierenden Änderungsgeometrie ein Übertragen der resultierenden Änderungsgeometrie auf ein zweites digitales dreidimensionales Zahnersatzteilmodells, wobei das zweite Zahnersatzteilmodell eine höhere Auflösung als das erste Zahnersatzteilmodell aufweist. Ausführungsformen können den Vorteil haben, dass sie ein mehrstufiges Modellieren eines patientenindividuellen Zahnersatzteils ermöglichen. Dabei nimmt die Genauigkeit des modellierten Zahnersatzteils von Stufe zu Stufe zu. So kann beispielsweise auf einer ersten Modellierungsstufe ein erstes digitales dreidimensionales Zahnersatzteilmodell mit einer ersten Auflösung verwendet werden. Bei dieser Auflösung kann es sich beispielsweise um eine vergleichsweise grobe Auflösung handeln, welche ein schnelles Anpassen grundsätzlicher Abmessungen des Zahnersatzteilmodells an die Gegebenheiten einer Patientensituationsgeometrie mit geringem Rechenaufwand ermöglichen. Die hierbei resultierende Änderungsgeometrie kann nun auf ein zweites Zahnersatzteilmodell mit einer höheren Auflösung als dem erste Zahnersatzteilmodell übertragen werden. Mit anderen Worten kann sich der Nutzer die bereits vorgenommenen Veränderungen zunutze machen. An dem zweiten Zahnersatzteilmodell kann sodann eine Feinanpassung an die entsprechenden Gegebenheiten der Patientensituationsgeometrie erfolgen.

Nach Ausführungsformen legen die geometrischen Anpassungskriterien ein oder mehrere zulässige Maximal- und/oder Minimalwerte für positive und/oder negative Abstände bzw. Offsets zwischen patientenspezifische Begrenzungsoberflächen des Patientensituationsmodells und zahnersatzteilspezifische Begrenzungsoberflächen des Zahnersatzteilmodells fest. Ausführungsformen können den Vorteil haben, dass durch die geometrischen Anpassungskriterien sichergestellt werden kann, dass jede der berechneten zustandsspezifischen Zustandsgeometrien des Zahnersatzteilmodells ein oder mehrere grundlegende geometrische Beziehungen in Bezug auf patientenspezifische Begrenzungsflächen erfüllt. Ein negativer Abstand bezeichnet einen Abstand zwischen einer patientenspezifische Begrenzungsoberfläche und eine zahnersatzteilspezifische Begrenzungsoberfläche, wobei sich die zahnersatzteilspezifische Begrenzungsoberfläche innerhalb des Patientensituationsmodells erstreckt. Ein positiver Abstand bezeichnet einen Abstand zwischen einer patientenspezifische Begrenzungsoberfläche und eine zahnersatzteilspezifische Begrenzungsoberfläche, wobei sich die zahnersatzteilspezifische Begrenzungsoberfläche außerhalb des Patientensituationsmodells erstreckt.

Nach Ausführungsformen handelt es sich bei den patientenspezifischen Begrenzungsflächen um Begrenzungsflächen eines oder mehrerer von dem Patientensituationsmodell umfassten Antagonisten, eines oder mehrerer von dem Patientensituationsmodell umfassten approximaler Zähne und/oder eines oder mehrerer von dem Patientensituationsmodell umfassten Zahnersatzteile. Nach Ausführungsformen betragen ein oder mehrere der zulässigen Maximal- und/oder Minimalwerte Null. Ausführungsformen können den Vorteil haben, dass eine Durchdringung von Begrenzungsflächen des Zahnersatzteilmodells und des Patientensituationsmodells grundsätzlich ausgeschlossen werden können.

Nach Ausführungsformen legen die geometrischen Anpassungskriterien ein oder mehrere zulässige Minimalwerte für positive Abstände zwischen zahnersatzteilspezifische Begrenzungsoberflächen des Zahnersatzteilmodells fest. Mit anderen Worten legen die die geometrischen Anpassungskriterien eine Mindestmaterialstärke für das Zahnersatzteilmodell fest.

Nach Ausführungsformen umfassen die unter Verwendung der patientenspezifischen Begrenzungsoberflächen definierten geometrischen Anpassungskriterien zumindest eines der folgenden von den im Zuge des patientenindividuellen Anpassens der Zahnersatzteilgeometrie berechneten zustandsspezifischen Zustandsgeometrien einzuhaltenden geometrischen Kriterien: von den Begrenzungsoberflächen begrenzte Materialstärken des Zahnersatzteilmodells unterschreiten nicht einen vordefinierten ersten Minimalwert, maximale Durchdringungstiefen von Begrenzungsflächen des Zahnersatzteilmodells und Begrenzungsflächen des Patientensituationsmodells überschreiten einen vordefinierten Maximalwert nicht, minimale Abstände zwischen Begrenzungsflächen des Zahnersatzteilmodells und Begrenzungsflächen des Patientensituationsmodells unterschreiten nicht einen vordefinierten zweiten Minimalwert.

Beispielsweise wird festgelegt, dass eine Materialstärke des Zahnersatzteilmodells einen vordefinierten Minimalwert nicht unterschreiten darf. Ausführungsformen können den Vorteil haben, dass so eine Langlebigkeit und hohe Stabilität des Zahnersatzteils sichergestellt werden kann. Insbesondere kann so auch sichergestellt werden, dass nicht Teile eines Objekts des Patientengebisses, wie beispielsweise eines Zahnstumpfs, infolge einer Verschiebung des Zahnersatzteilmodells dasselbe vollständig durchstoßen und teilweise aus diesem herausragen. Falls sich ein Abstand zwischen zwei Begrenzungsflächen infolge benutzerdefinierter Veränderungen soweit reduziert, dass dieser dem vordefinierten ersten Minimalwert entspricht, so kann eine weitere Annäherung der entsprechenden Begrenzungsflächen unterbunden werden. Vielmehr wird der Abstand auf dem entsprechenden Minimalwert konstant gehalten und weitere Veränderungen erfolgen durch eine geometrische Verformung des Zahnersatzteilmodells. Möchte ein Nutzer beispielsweise das Zahnersatzteilmodell innerhalb der Patientensituationsgeometrie verschieben, so kann sich beispielsweise eine Situation einstellen, in der zwar die Krone des Zahnersatzteilmodells weiter verschoben wird, ein unterer Abschnitt des Zahnersatzteilmodells aber ortsfest bleibt bzw. Teilbereiche von Begrenzungsflächen des unteren Abschnitts ortsfest bleiben.

Nach Ausführungsformen handelt es sich bei den Begrenzungsflächen des Patientensituationsmodells für welche die maximale Durchdringungstiefen oder minimalen Abstände begrenzt sind um Begrenzungsflächen eines oder mehrerer von dem Patientensituationsmodell umfassten Antagonisten, eines oder mehrerer von dem Patientensituationsmodell umfassten approximaler Zähne und/oder eines oder mehrerer von dem Patientensituationsmodell umfassten Zahnersatzteile.

Beispielsweise kann festgelegt werden, dass maximale Durchdringungstiefen von Begrenzungsflächen des Zahnersatzteilmodells und Begrenzungsflächen des Patientensituationsmodells einen vordefinierten Maximalwert nicht überschreiten. Hierbei kann beispielsweise festgelegt werden, dass keine Durchdringung zulässig ist oder, falls eine entsprechende Durchdringung zulässig ist, kann diese auf einen zulässigen Maximalwert beschränkt werden. Eine begrenzte Durchdringung zwischen Zahnersatzteilmodell und Patientensituationsmodell kann beispielsweise zulässig sein, zwischen dem Zahnersatzteilmodell und einem Antagonisten. Hierbei kann eine minimale Durchdringung sinnvoll sein, um ein Kontaktgefühl des Patienten beim Beißen und ein effektives Kauen sicherstellen zu können. Ferner kann eine unbegrenzte Durchdringung im Zuge des Modellierens sinnvoll sein, um zunächst das Zahnersatzteilmodell als Ganzes an eine Patientensituationsgeometrie anzupassen und verbleibende minimale Durchdringungen im Zuge einer Feinanpassung am Ende des Modellierungsverfahrens lokal zu beheben. Dies kann beispielsweise durch eine lokale Verformung der Oberflächengeometrie des Zahnersatzteilmodells oder durch ein lokales Abtragen von Material des Zahnersatzteilmodells erfolgen.

Beispielsweise kann festgelegt werden, dass maximale Abstände zwischen Begrenzungsflächen des Zahnersatzteilmodells und Begrenzungsflächen des Patientensituationsmodells einen vordefinierten Maximalwert nicht überschreiten. Ausführungsformen können den Vorteil haben, dass so sichergestellt werden kann, dass Abstände zwischen Zahnersatzteilmodell und Patientensituationsmodell abschnittsweise nicht zu groß werden.

Beispielsweise kann festgelegt werden, dass minimale Abstände zwischen Begrenzungsflächen des Zahnersatzteilmodells und Begrenzungsflächen des Patientensituationsmodells einen vordefinierten Minimalwert nicht unterschreiten. Ausführungsformen können somit den Vorteil haben, dass ein Abstand zwischen Zahnersatzteilmodell und Patientensituationsmodell abschnittsweise nicht zu gering ausgestaltet werden kann.

Wird ein durch eines der Anpassungskriterien festgelegter Grenzwert erreicht, so kann beispielsweise definiert sein, dass weitere benutzerindividuelle Veränderungen des Zahnersatzteilmodells durch ein Verformen desselben unter der Maxime ermöglicht wird, dass die Anpassungskriterien eingehalten werden.

Ausführungsformen können den Vorteil haben, dass eine Durchdringung von Begrenzungsflächen des Zahnersatzteilmodells und des Patientensituationsmodells grundsätzlich ausgeschlossen werden können.

Nach Ausführungsformen sind die geometrischen Anpassungskriterien hierarchisch gegliedert. Im Falle nicht miteinander vereinbarer geometrischer Anpassungskriterien bekommen einzelne geometrische Anpassungskriterien gemäß der hierarchischen Gliederung Vorrang gegenüber ein oder mehreren anderen geometrischen Anpassungskriterien gewährt. Ausführungsformen können den Vorteil haben, dass sich Konflikte zwischen geometrischen Anpassungskriterien anhand der hierarchischen Gliederung lösen lassen. Beispielsweise kann das Auswahlkriterium eines Beibehaltens eines Mindestwerts für die Materialstärke höher gewichtet werden als das Anpassungskriterium einer Begrenzung der Durchdringungstiefe. Beispielsweise kann bei einem Widerspruch zwischen zwei Anpassungskriterien das hierarchisch niedriger angeordnete Anpassungskriterium aufgeweicht werden, so kann beispielsweise im Falle eines Widerspruchs zwischen minimaler Materialstärke und maximal zulässiger Durchdringungstiefe die maximal zulässige Durchdringungstiefe erhöht werden. Ein infolge eines solchen Konflikts verletztes Anpassungskriterium lässt sich beispielsweise im Zuge einer Feinpassung des Zahnersatzteilmodells am Ende jedes Modellierungsvorgangs beheben. So kann beispielsweise eine gewünschte Durchdringung durch ein lokales Verformen oder Materialabtragens an dem Zahnersatzteilmodell gelöst werden.

Nach Ausführungsformen erfolgt die das Anzeigen der benutzerdefinierten Veränderung jeweils in Echtzeit erfolgt. Ausführungsformen können den Vorteil haben, dass ein Nutzer direkt sieht, welche Auswirkungen benutzerdefinierte Veränderungen haben und diese schnell und effektiv anpassen kann.

Nach Ausführungsformen umfassen die benutzerdefinierten Veränderungen jeweils zumindest eine der folgenden durch eine interaktive Benutzereingabe definierten Veränderungen: ein Skalieren einer Erstreckung des Zahnersatzteilmodells entlang einer vordefinierten Erstreckungsrichtung des Zahnersatzteilmodells, ein Verschieben des Zahnersatzteilmodells relativ zu dem Patientensituationsmodell und ein Rotieren des Zahnersatzteilmodells relativ zu dem Patientensituationsmodell.

Nach Ausführungsformen umfasst die Eingabe der benutzerdefinierten Veränderungen jeweils ein Selektieren und interaktives Bearbeiten mindestens eines Teilbereichs einer Begrenzungsoberfläche des auf der graphischen Benutzeroberfläche visuell wiedergegebenen Zahnersatzteilmodells mittels eines von der graphischen Benutzeroberfläche bereitgestellten interaktiven digitalen Bearbeitungswerkzeugs .

Nach Ausführungsformen umfasst das interaktive Bearbeiten ein Verformen eines Teilbereichs und/oder ein Abschneiden eines von dem Teilbereich begrenzten Volumenabschnitts der Zahnersatzteilgeometrie.

Ein Verformen kann den Vorteil haben, dass eine generelle Form des Zahnersatzteils möglichst weitgehend erhalten bleibt. Ausführungsformen können den Vorteil haben, dass lokale Anpassungen vorgenommen werden können, während die generelle Zahnform unverändert beibehalten werden kann. Insbesondere können so glatte Übergänge zwischen unterschiedlichen Teilbereichen der Begrenzungsoberflächen sichergestellt werden.

Ein Abschneiden einer Begrenzungsoberfläche, ggf. mit Offset, kann den Vorteil haben, dass das Zahnersatzteil außerhalb des Abgeschnittenen Teilbereiches möglichst wenig oder gar nicht geändert wird. So können beispielsweise sogenannte Facettenflächen bzw. Schliffflächen auf Zähnen erhalten werden. Ausführungsformen können den Vorteil haben, dass lokale Anpassungen des Zahnersatzteilmodells ermöglicht werden, während die restliche Geometrie möglichst unverändert beibehalten werden kann.

Durch ein lokales Abschneiden und/oder Verformen können beispielsweise berührungskontakte, z.B. approximal und/oder okklusal, optimiert werden. So lässt sich beispielsweise ein Bisskontakt einer okklusalen Begrenzungsoberfläche des Zahnersatzteilmodells mit okklusalen Begrenzungsoberfläche eines von dem Patientensituationsmodell umfassten Antagonisten des Zahnersatzteil optimieren.

Nach Ausführungsformen erfolgt die Eingabe der benutzerspezifischen Veränderungen unter Verwendung einer Eingabevorrichtung. Die Eingabevorrichtung umfasst beispielsweise eine Maus, eine Tastatur, ein Touchpad, einen Touchscreen, einen Eingabestift bzw. Stylus, eine dreidimensionale haptische Eingabevorrichtung und/oder eine Vorrichtung zur Gestenerkennung. Nach Ausführungsformen wird die Eingabevorrichtung dazu verwendet das interaktive Bearbeitungswerkzeug auf der graphischen Benutzeroberfläche zu steuern. Nach Ausführungsformen umfasst das interaktive digitale Bearbeitungswerkzeug einen Zeiger zum Selektieren eines Teilbereichs einer Oberfläche und Verschieben des selektierten Teilbereichs auf der graphischen Benutzeroberfläche. Das Verschieben kann beispielsweise als Dragand-drop-Funktion ausgeführt werden. Der Zeiger kann eine beliebige Ausgestaltung aufweisen, insbesondere kann er als ein Werkzeug ausgestaltet sein, welches die durch den Zeiger bzw. das interaktive digitale Bearbeitungswerkzeug bereitgestellte Bearbeitungsmöglichkeiten symbolisiert. Nach Ausführungsformen umfasst das Bearbeiten ein lokales Auftragen von Material, Abtragen von Material, Glätten von Fissuren und/oder Vertiefen von Fissuren. Nach Ausführungsformen wird ein Punkt der Oberfläche selektiert. Nach Ausführungsformen wird der Teilbereich mittels Pinselfunktion selektiert.

Nach Ausführungsformen sind in dem digitalen dreidimensionalen Zahnersatzteilmodell ein oder mehrere Teilbereiche funktionsspezifisch definiert. Teilbereiche umfassen beispielsweise Höcker, Höckerspitze, Fissuren, Kauflächen, etc. Nach Ausführungsformen ist für ein oder mehrere der funktionsspezifisch definierten Teilbereiche vordefiniert, ob diese veränderbar, z.B. beweglich, oder unveränderbar sind, z.B. in ihrer ursprünglichen Form und/oder Position beizubehalten sind. Nach Ausführungsformen kann der Nutzer beispielsweise über eine Nutzereingabe oder Hotkeys festlegen, ob funktionsspezifisch definierten Teilbereiche veränderbar und/oder in welchem Umfang die entsprechenden Teilbereiche veränderbar sind oder ob die entsprechenden Teilbereiche unveränderbar sind.

Nach Ausführungsformen erfolgt eine Eingabe der benutzerdefinierten Veränderung indirekt über Hotkeys, Zahlenwerte oder sonstige 2D-Kontrollelemente, wobei ein zwischengeschalter Algorithmus abhängig von der Eingabe eine Veränderung des digitalen dreidimensionalen Zahnersatzteilmodells ausführt. Eine Veränderung kann beispielsweise eine Veränderung eines Artikulatorzustands, ein Schrumpfen, ein Aufblähen und/oder eine Zahnalterung bzw. Zahnabflachung umfassen.

Nach Ausführungsformen umfasst die Eingabe der benutzerdefinierten Veränderungen ein Einstellen eines einstellbaren Materialauftragswerts, um auf selektierten Teilbereich des ersten Zahnersatzteilmodells, beispielswiese eine Krone oder einen Teilbereich der Krone, virtuell Material auf- oder abzutragen. Nach Ausführungsformen kann der Materialauftragswert positiv sein, im Fall einer Materialzunahmen, oder negativ, im Fall einer Materialabnahme.

Ausführungsformen können den Vorteil haben, dass ein Materialabtragen und/oder Materialauftragen in Echtzeit ausgeführt und durch den Nutzer beurteilt werden kann. Somit lässt sich beispielsweise eine notwendige Schichtdicke für eine auf das herzustellende Zahnersatzteil aufzubringende Keramikverblendung schnell abzuschätzen.

Ein Materialabtragen bzw. Schrumpfen und Materialauftragen bzw. Aufblähen entspricht technisch nicht ganz einem Skalieren. Vielmehr handelt es sich hierbei um ein Bewegen eines Teilbereichs der Oberfläche des Zahnersatzteilmodells um einen über den Teilbereich, d.h. für jeden Punkt des Teilbereichs, konstanten Abstandswert relativ zu einem Zentrum des Zahnersatzteilmodells. Dabei ist der konstante Abstandswert unabhängig vom Abstand zum Zentrum.

Nach Ausführungsformen werden mittels Materialabtragen und/oder Materialauftragen vordefinierter Teilbereiche des Zahnersatzteilmodells verändert. Beispielsweise handelt es sich bei diesen Teilbereichen um ästhetisch relevante Teilbereiche, welche, z.B. mit Keramik, zu verblenden sind. Ästhetisch relevante Teilbereiche sind Teilbereiche Zahnersatzteilmodells, welche Teilbereichen des herzustellenden Zahnersatzteils entsprechen, die bei einem Anordnenden des Zahnersatzteils in dem Patientengebiss bei üblichen Mundbewegungen regelmäßig sichtbar sind.

Nach Ausführungsformen umfasst das Anordnen des Zahnersatzteilmodells in der Ausgangsposition eine automatische Anpassung des Zahnersatzteilmodells an eine in dem Patientensituationsmodell definierte Präparationsgrenze für das Zahnersatzteil. Ausführungsformen können den Vorteil haben, dass durch das Patientensituationsmodell vorgegebene Präparationsgrenzen stets eingehalten werden. Somit kann insbesondere verhindert werden, dass infolge einer benutzerdefinierten Veränderung des Zahnersatzteilmodells eine Nachbesserung zum Einhalten der Präparationsgrenze notwendig wird. Eine entsprechende Präparationsgrenze kann beispielsweise durch den Nutzer zu Beginn des Verfahrens festgelegt und an patientenindividuelle Gegebenheiten des Patientensituationsmodells angepasst werden.

Nach Ausführungsformen umfasst das Verfahren ferner:
- Auswählen eines Änderungszustands des ersten Zahnersatzteilmodells,
- Simulieren einer Kaubewegung für den ausgewählten Änderungszustands des ersten Zahnersatzteilmodells, wobei das Simulieren der Kaubewegung eine Berechnung einer dynamisch durchlaufenen Sequenz von relativen Positionen des Zahnersatzteilmodells zu einem von dem Patientensituationsmodell umfasste Antagonisten des Zahnersatzteilmodells umfasst, wobei für jede der relativen Positionen mittels der grafischen Benutzeroberfläche auf der Anzeigevorrichtung jeweils zumindest eine okklusale Begrenzungsfläche des Zahnersatzteilmodell und eine okklusale Begrenzungsfläche des Antagonisten angezeigt wird.

Ausführungsformen können den Vorteil haben, dass ein virtueller Artikulator bereitgestellt wird, mittels welchem die Auswirkungen von Anpassungen des Zahnersatzteilmodells auf dynamische Kaubewegungen des Pateienten simuliert werden können. Somit lässt sich sicherstellen, dass eine Anpassung des Zahnersatzteils nicht nur für eine einzelne relative Positionierung gegenüber einem Antagonisten, sondern für relative Positionierung im Zuge einer Kaubewegung passt.

Nach Ausführungsformen werden für die einzelnen relativen Positionen der dynamischen Sequenz jeweils Teilbereiche der okklusalen Begrenzungsfläche des Zahnersatzteilmodells angezeigt, welche die okklusale Begrenzungsfläche des Antagonisten durchdringen. Nach Ausführungsformen handelt es sich bei der Kaubewegung um eine generische Kaubewegung. Nach Ausführungsformen handelt es sich bei der Kaubewegung um patientenindividuelle Kaubewegung.

Im Zuge des Simulierens der Kaubewegung wird beispielsweise der Antagonist in seiner relativen Position zu dem Zahnersatzteil beeinflusst, erweitert und/oder modifiziert. Beispielsweise werden im Zuge der Simulation eine oder mehrere vorbestimmte Positionen, welche sich nicht überlagern, mit Bewegungsbahnen angefahren und an den entsprechenden vorbestimmten Positionen als Lage des Antagonisten angepasst. Das ist beispielsweise der Fall, wenn ein Nutzer eine vorher vom virtuellen Artikulator berechnete Kieferbewegungsposition per Slider auswählt und dann direkt ein Ergebnis für die entsprechende Position angezeigt bekommt.

Beispielsweise wird im Zuge der Simulation ein neuer virtueller Antagonist als Überlagerung der vieler durch die Bewegungsbahnen zusammengefassten Antagonistenpositionen erzeugt und jeweils in einzelnen Bewegungen des Gegenbisses als Virtual-Imprint dargestellt. In diesem Fall wird eine neue künstliche Begrenzungsfläche aus einer Hüllfläche aller Kieferbewegungsbahnen erzeugt.

Ein virtueller Artikulator bezeichnet eine vereinfachte digitale Simulation eines realen Artikulators, welcher ein zahntechnisches Gerät zur Simulation einer Kaubewegung mit üblichen Bewegungsabläufen, z.B. Laterotrusion terosion links/rechts, Retrusion etc. darstellt. Alternativ können auch Bewegungsbahnen des Kiefers aus an dem Patienten messenden Systemen eingeladen werden und für die Bewegung des Antagonisten genutzt werden.

Ausführungsformen können den Vorteil haben, dass ein dynamisches Wechselspiel des Zahnersatzteilmodells mit Antagonisten in der Patientensituationsgeometrie im Zuge des Kauens berücksichtigt werden können. Insbesondere kann somit ein Anpassen des Zahnersatzteilmodells an unterschiedliche Wechselwirkungssituationen erfolgen.

Nach Ausführungsformen handelt es sich bei dem bereitgestellten Zahnersatzteilmodell in dem Ausgangszustand um ein generisches Modell für das Zahnersatzteil. Ausführungsformen können den Vorteil haben, dass generische Modelle verwendet werden können, welche grundsätzliche geometrischen Bedingungen, wie beispielsweise Abstandsverhältnisse zwischen Punkten des Zahnersatzteilmodells, festlegen. Im Zuge des Modellierens kann ein entsprechendes generisches Modell an die Gegebenheiten eines patientenindividuellen Patientensituationsmodells angepasst werden. Hierbei können die generischen Gegebenheiten des zugrunde liegenden generischen Modells beispielsweise beibehalten werden. Entsprechende generische Zahnersatzteilmodelle werden beispielsweise in Form von Bibliotheken bereitgestellt, aus welche diese geladen werden können.

Nach Ausführungsformen umfasst das Bereitstellen des Zahnersatzteilmodell in dem Ausgangszustand ein Auswählen und Kopieren eines von dem Patientensituationsmodell umfassten Zahns und/oder Zahnteils. Nach Ausführungsformen umfasst das Bereitstellen ferner ein Spiegeln. Ausführungsformen können den Vorteil haben, dass zum Modellieren eines Zahnersatzteils bereits von einem Zahnersatzteilmodell ausgegangen werden kann, welches zumindest teilweise an patientenindividuelle Gegebenheiten des Patientensituationsmodells angepasst ist. Ein solches bereits vorangepasstes Zahnersatzteilmodell kann den Vorteil haben, dass dadurch der Anpassungsprozess verkürzt wird. Ausführungsformen können den Vorteil haben, dass auf bereits vorhandene patientenindividuelle Objekte zurückgegriffen werden kann.

Nach Ausführungsformen umfasst das Bereitstellen des Zahnersatzteilmodell in dem Ausgangszustand ein Auswählen und Kopieren eines an das Patientensituationsmodell bereits angepassten patientenindividuellen Zahnersatzteils. Nach Ausführungsformen umfasst das Bereitstellen ferner ein Spiegeln. Ausführungsformen können den Vorteil haben, dass auf bereits angepasste patientenindividuelle Zahnersatzteile zurückgegriffen werden kann.

Nach Ausführungsformen umfassen die Objekte eines Patientengebisses ein oder mehrere der folgenden Objekte: einen Zahn, einen Zahnstumpf, Zahnfleisch, einen Zahnersatz, ein Implantat, einen Zahnhalteapparat, einen Locator, eine Aufbissschiene, ein Steg, eine Prothese oder eine Teilprothese, eine Modellgussprothese, ein Zahnersatzprovisorium, Zahnfüllung, Inlay. Ein Zahnhalteapparat bezeichnet ein funktionelles Verankerungssystem eines Zahnes, für welches auch der Begriff Attachment verwendet wird. Ein Locator bezeichnet ein konfektioniertes Verbindungselement zur Verbindung eines herausnehmbaren Zahnersatzes mit einem Implantat. Eine Aufbissschiene bezeichnet eine an den Zahnbogen angepasste prothesenähnliche Auflage, insbesondere aus Kunststoff, welche beispielsweise zur Behandlung von Myoarthropathien verwendet wird. Ein Steg wird beispielsweise zu Befestigung einer Stegprothese verwendet. Dabei stellt der Steg hat eine Halte- und/oder Stützfunktion zum Halten und/oder Stützen der Stegprothese bereit. Ein solcher Steg kann beispielsweise einen runden oder rechteckigen Querschnitt aufweisen. Ein Steg dient zum Verblocken mindestens zweier Zähne oder Implantate, wobei der Steg beispielsweise an Wurzelkappen, Ankerkronen oder Suprastrukturen befestigt sein kann. Beispielsweise können mehrere Stege miteinander verbunden werden. Nach Ausführungsformen umfasst das Verfahren ferner ein Herstellen des patientenindividuellen Zahnersatzteils unter Verwendung der als patientenindividuellen Zahnersatzteilgeometrie festgelegten Änderungsgeometrie.

Nach Ausführungsformen sind die patientenspezifischen und zahnersatzteilspezifischen Begrenzungsoberflächen unter Verwendung eines der folgend Verfahren implementiert sind: einer polygonalen Netzstruktur, wobei Vertices der entsprechenden Netzstruktur und/oder Punkte innerhalb der Polygone der Netzstruktur die entsprechenden Begrenzungsoberflächen definieren, einer Punktewolke, wobei die Punkte der Punktewolke die entsprechenden Begrenzungsoberflächen definieren, einer 3D-Volumendatenstruktur, welche ein Voxelgitter umfasst, oder eines 3D-Signed-Distance-Fields.

Nach Ausführungsformen wird die Anzahl und Schrittweite der dynamischen Sequenz abhängig von einer gewünschten Zielframerate für die angezeigte Animation oder abhängig von einer visuell zu erwartenden Änderung bestimmt. So kann vermieden werden den Computer nicht unnötig zu belasten und zugleich eine bestmögliche Interaktivität gewährleisten werden. Beispielsweise können, um eine Bewegung zu beschleunigen, weniger Zwischenschritte berechnet werden. Umgekehrt können, um eine Bewegung für eine Feinabstimmung zu beschleunigen, mehr Zwischenschritte berechnet werden.

Ausführungsformen können den Vorteil haben, dass einerseits ein hohes Maß an Interaktivität gewährleistet werden kann, während andererseits ein computerseitiger Rechenaufwand reduziert werden kann. Zudem kann durch eine Reduktion der Anzahl der Zwischenschritte ein Durchlaufen der dynamischen Sequenz erhöht werden, wodurch ein schnelleres Anpassen des Zahnersatzteilmodells ermöglicht werden kann.

Nach Ausführungsformen werden vordefinierte Bereiche des Zahnersatzteilmodells außerhalb einer Grenzfläche gehalten. Entsprechende Grenzflächen sind beispielsweise vordefiniert oder können von dem Nutzer eingestellt werden. Ausführungsformen können den Vorteil haben, dass lediglich ein größenmäßig beschränkter Übergangsbereich außerhalb der vordefinierten Bereiche elastisch verformt wird. Um beispielsweise an einer Zahnhöckerspitze ziehen zu können bis diese an einer passenden Position in der Gegenkieferkaufläche angeordnet ist, wird der Zahnhöcker selbst dabei nicht zu stark zu verformen, wenn es sich bei diesem um einen entsprechenden vordefinierte Bereiche handelt.

Nach Ausführungsformen beeinflussen sich im selben Kiefer oder in gegenüberliegenden Kiefer benachbart angeordnete Zahnersatzteilmodelle gegenseitig, um eine zahntechnisch optimale Form zu erhalten. Hierbei kann beispielswiese der Oberkiefer Priorität zum dem Unterkiefer erhalten und vorne im Gebissanzuordnende Zahnersatzteile können aus ästhetischen Gründen Priorität gegenüber weiter hinten anzuordnenden Zahnersatzteilen haben. Beispielsweise kann es Mischformen geben, bei welchen die Priorität prozentual definiert ist. Dabei kann festgelegt werden, welches der beiden sich beeinflussenden Zahnersatzteile mehr erhalten werden soll und welche weniger. Ausführungsformen können den Vorteil haben, dass durch ein Festlegen von Prioritäten Konflikte zwischen einander gegenseitig beeinflussenden Anpassungen von Zahnersatzteilemodellen aufgelöst werden können.

Nach Ausführungsformen werden ein oder mehrere vom Nutzer gewählte Zahnersatzteile auf einer Begrenzungsoberfläche des Patientenmodells, ggf. mit Offset, festgehalten oder daran angeschmiegt. So kann beispielsweise eine Kontaktflächen zwischen einem Zahnersatzteil und einem Antagonisten an bestimmten Stellen erzwungen werden. Gleichzeitig können optional andere Zahnersatzteile außer Kontakt zur selben Begrenzungsoberfläche, ggf. mit einem Offset gebracht werden, z.B. um genug Abstand für die Höckerspitzen oder Fissuren des entsprechenden Zahnersatzteiles zum Antagonisten zu lassen. Ausführungsformen können den Vorteil haben, dass unterschiedliche Anpassungserfordernisse an unterschiedliche Teilbereiche eines Zahnersatzteilmodells und/oder unterschiedliche Zahnersatzteilmodell erfüllt werden können.

Nach Ausführungsformen können Grenzflächen für patientenspezifische und/oder zahnersatzteilspezifischen Begrenzungsoberflächen vom Nutzer über geometrische erzeugte Objekte definiert werden. Zum Beispiel kann eine digitale Grenzfläche in Form einer Ebene eingeladen und platziert werden, etwa in Form einer Spee-Wilson Kurvenflächen. Ferner eine digitale Grenzfläche z.B. aus einer Bibliothek eingeladen werden. Beispielsweise wird eine Kalotte aus einer Bibliothek eingeladen und Platziert. Eine Kalotte dient als Aufstellhilfe für unbezahnte Kiefer oder für vorgefertigten Zahnaufstellungen für den Gegenkiefer, was etwa in der Totalprothetik vorteilhaft sein kann. Ausführungsformen können den Vorteil haben, dass durch diese das Modellieren effizienter und effektiver gestaltet werden kann. Insbesondere kann ein Ergänzen zusätzlicher Elemente vereinfacht werden.

Nach Ausführungsformen wird eine der patientenspezifischen Begrenzungsoberfläche als Mindestdickenfläche zu einer patientenspezifische Begrenzungsoberflächen definiert, etwa eines Zahnstumpfs, Zahnimplantatteils, und/oder Zahnabutmentteils. Hierbei kann beispielsweise eine Offsetfläche auf einem Zahnstumpf definiert werden, es kann eine Mindestdickenoberfläche eingeladen oder optional darauf ein materialspezifisch generierte Offsetfläche festgelegt werden.

Nach Ausführungsformen wird eine physikalisch vorhandene Form absolut berücksichtigt und bei Überschreitung der Formgebung on-the-fly angepasst. Die Anpassung wird beispielsweise durch Abschneiden und/oder unter Berücksichtigung einer vordefinierten Morphologie, wie etwa einer Zahnmorphologie, an eine zur Verfügung stehenden freien Raum innerhalb der physikalisch vorgegebenen Form hergestellt. Dies kann beispielsweise vorteilhaft sein für Totaprothetikzähne. Nach Ausführungsformen kann dies umgesetzt werden, indem pro Zahnersatzteil eine zusätzliche Fläche eingeladen, stets mit dem Zahnersatzteil bzw. relativ zum Zahnersatzteil mitbewegt wird und als Begrenzung der aktuell modellierten Zahnersatzteilgeometrie dient. Eingeladen werden kann eine entsprechende Fläche beispielsweise aus einer Pre-Form-Bibliothek, d.h. beispielsweise einer Bibliothek von Formen für die Verwendung von Rohlingen mit vorgefertigtem Implantatanschluss, oder einer Totalprothetikzahn-Bibliothek.

Ausführungsformen können den Vorteil haben, dass auch Pre-Formen im Wechselspiel mit dem Zahnersatzteilmodell effektiv und effizient berücksichtigt werden können. Ausführungsformen können den Vorteil haben, dass Änderungen in der Bibliothek effektiv und effizient implementiert werden können. Insbesondere können Ausführungsformen sicherstellen, dass eine bereits erfolgte Anpassung übernommen werden kann und kein erneutes Beginnen von null notwendig ist.

Nach Ausführungsformen kann ein Wechsel einer Bibliothek durchgeführt werden, wobei ein erstes Zahnersatzteilmodell aus einer ersten Bibliothek gegen ein zweites Zahnersatzteilmodell aus einer zweiten Bibliothek ausgetauscht wird. Nach Ausführungsformen wird dabei eine aktuelle Änderungsgeometrie des ersten Zahnersatzteilmodells auf das zweite Zahnersatzteilmodell übertragen. Dies kann beispielsweise eine Positionierung sowie Form, z. B. Lage von Höckern, Fissuren, oder sonstigen Kontaktpunkte, des ersten Zahnersatzteilmodells umfassen. Ausführungsformen können den Vorteil haben, dass bei einem Bibliothekswechsel nicht alle Anpassungen erneut durchgeführt werden müssen.

Nach Ausführungsformen werden mehrere Zahnersatzteilmodelle aus unterschiedlichen Bibliotheken für dasselbe Zahnersatzteil mittel der graphischen Benutzeroberfläche dargestellt. Ausführungsformen können den Vorteil haben, dass für dasselbe Zahnersatzteil gleichzeitig Zahnersatzteilmodelle aus mehreren dargestellt und on-the-fly mitbewegt bzw. mitverändert werden können. Ein Anpassen und Darstellen kann dabei entweder per Splitscreen Darstellung oder gleichzeitig in Form einer Überlagerung, z. B. transparent und/oder mit verschiedenen Farben, erfolgen.

Ausführungsformen können den Vorteil haben, dass unterschiedliche Bibliotheken simultan berücksichtigt werden können. Dies ermöglicht es, ein und dieselbe Anpassung für mehrere Bibliotheken gleichzeitig auszuführen, sodass am Ende identische Anpassungsergebnisse für unterschiedliche Bibliotheken erzeugt werden können und eine Auswahl aus diesen erfolgen kann. Insbesondere kann so eine Anpassung erfolgen, welche nicht nur für eine, sondern für mehrere und/oder alle dargestellten Bibliotheken zugleich optimiert ist.

Nach Ausführungsformen handelt es sich bei dem Zahnersatzteil um eine Brücke, welche beispielsweise aus einer Brücken-Bibliotheken geladen wird. Ausführungsformen können den Vorteilhaben, dass eine relative Lage und/oder Größe einzelner oder mehrerer von der Brücke umfasster Zähne fest vorgegeben ist. Dies kann beispielsweise für eine Totalprothetik von Backenzähnen vorteilhaft sein. Nach Ausführungsformen werden diese Backenzähne gemeinsam eingeladen und sind in der Größe und Lage festzueinander festgelegt. Nach Ausführungsformen sind ganze Zahnbrücken gemeinsam als eine Oberfläche hinterlegt sind.

Ausführungsformen können den Vorteil haben, dass im Falle von Brücken und/oder Brückenteile eine Anpassung vereinfacht und beschleunigt werden kann, da diese als ein Objekt bzw. ein Zahnersatzteilmodell bereitgestellt und angepasst werden.

Nach Ausführungsformen umfasst das Zahnersatzteilmodell, welches z.B. aus einer Bibliothek geladen wird, einen Gingiva-Anteil. Ausführungsformen können den Vorteilhaben, das ein Gingiva-Anteil direkt in dem Zahnersatzteilmodel mitenthalten ist und z.B. mit einer Änderung einer Zahnform des Zahnersatzteilmodells mitverformt und/oder mitbewegt werden kann. Nach Ausführungsformen handelt es sich bei dem Zahnersatzteilmodell mit Gingiva-Anteil um ein Brückenmodell, welches etwa aus einer Brückenbibliotheken geladen wird.

Nach Ausführung formen wird ein digitaler Gingiva-Anteile automatisch, z.B. on-the-fly, für ein gegebenes Zahnersatzteilmodell erzeugen. Beispielsweise wird ein Gingiva-Anteile dort erzeugt, wo zu viel Abstand zwischen approximalen Begrenzungsflächen ist oder wo zu viel Abstand zu einem Patientenkiefer ist. Ausführungsformen können den Vorteil haben, dass Zahnfleischlücken verhindert bzw. automatisch aufgefüllt werden können. Ausführungsformen können den Vorteil haben, dass auch ein Gingiva-Anteil effektiv und effizient berücksichtigt werden kann bei der Anpassung von Zahnersatzteilmodellen.

Nach Ausführungsformen erfolgen Transformationen und Deformationen von Frontzähnen symmetrisch. Beispielsweise werden Transformationen und Deformationen symmetrisch gespiegelt. Ausführungsformen können den Vorteil haben, dass für Frontzähne eine symmetrische Anpassung implementiert werden kann.

Nach Ausführungsformen umfasst das Verfahren ein Simulieren einer Zahnalterung bzw. Quasi-Abrasion. Mit zunehmendem Alterungsgrad werden beispielsweise Fissuren zunehmend erhöht, Zahnhöcker zunehmend abgesenkt und/oder Kauflächen zunehmend abgeflacht. Eine Eingabe des alterungsgrad kann beispielsweise als benutzerdefinierten Veränderung über einen einstellbaren Wert erfolgen. Hierdurch kann ein heuristisch simulierter Zahnalterungsprozess, z.B. on-the-fly, gesteuert werden. Ausführungsformen können den Vorteil haben, dass Alterungsprozesse effektiv und effizient im Zuge des Modellierens berücksichtigt werden können.

Die Erfindung umfasst ferner ein Computerprogrammprodukt zum Modellieren eines patientenindividuellen Zahnersatzteils, welches ein nichtflüchtiges computerlesbares Speichermedium mit computerlesbaren Programminstruktionen zum Modellieren des patientenindividuellen Zahnersatzteils umfasst, wobei ein Ausführen der Programminstruktionen durch einen Prozessor eines Computersystems das Computersystem dazu veranlasst ein Verfahren zum Modellieren des patientenindividuellen Zahnersatzteils auszuführen, welches umfasst:
- Bereitstellen eines digitalen dreidimensionalen Patientensituationsmodells, wobei das Patientensituationsmodell patientenspezifische Begrenzungsoberflächen von ein oder mehreren Objekten eines Patientengebisses definiert, welche eine Patientensituationsgeometrie definieren, an welche das Zahnersatzteil im Zuge der Modellierung anzupassen ist,
- Bereitstellen eines digitalen dreidimensionalen Zahnersatzteilmodells in einem Ausgangszustand, wobei das Zahnersatzteilmodell zahnersatzteilspezifische Begrenzungsoberflächen des Zahnersatzteils definiert, welche eine Zahnersatzteilgeometrie definieren,
   wobei das Zahnersatzteilmodell in dem Ausgangszustand eine Zahnersatzteilgeometrie in Form einer Ausgangsgeometrie aufweist,
- Bereitstellen ein oder mehrere unter Verwendung der patientenspezifischen Begrenzungsoberflächen definierten geometrischen Anpassungskriterien, welches durch die zahnersatzteilspezifische Begrenzungsoberflächen im Zuge eines patientenindividuellen Anpassens der Zahnersatzteilgeometrie an die Patientensituationsgeometrie einzuhalten sind,
- patientenindividuelles Anpassen der Zahnersatzteilgeometrie des Zahnersatzteilmodells an die Patientensituationsgeometrie des Patientensituationsmodells,
   wobei das patientenindividuelle Anpassen ein Anordnen des Zahnersatzteilmodells in einer von dem Patientensituationsmodell für das Zahnersatzteil bereitgestellten Ausgangsposition umfasst,
   wobei das patientenindividuelle Anpassen ferner ein wiederholtes interaktives Ausführen benutzerdefinierter Veränderungen an dem angeordneten Zahnersatzteilmodell umfasst, wobei das Zahnersatzteilmodell im Zuge jeder der benutzerdefinierten Veränderungen jeweils dynamisch eine Sequenz von Zwischenzuständen durchläuft bis ein aus der jeweiligen benutzerdefinierten Veränderung resultierender Änderungszustand erreicht wird, wobei für jeden der entsprechenden Zwischenzustände sowie den resultierenden Änderungszustand jeweils automatisch aus der Ausgangsgeometrie des Zahnersatzteilmodells eine zustandsspezifische Zustandsgeometrie des Zahnersatzteilmodells unter Einhaltung der geometrischen Anpassungskriterien berechnet wird,
   wobei jede der benutzerdefinierten Veränderungen mittels einer grafischen Benutzeroberfläche auf einer Anzeigevorrichtung angezeigt wird, wobei das Anzeigen der jeweiligen benutzerdefinierten Veränderung jeweils ein Anzeigen des dynamischen Durchlaufens der jeweiligen Sequenz von Zwischenzuständen bis zum Erreichen des jeweiligen Änderungszustands durch das Zahnersatzteilmodell mit den jeweiligen hierfür berechneten zustandsspezifische Zustandsgeometrien umfasst,
- Festlegen einer aus dem patientenindividuellen Anpassen des Zahnersatzteilmodells resultierenden Änderungsgeometrie als zum Herstellen des patientenindividuellen Zahnersatzteils zu verwendende patientenindividuellen Zahnersatzteilgeometrie.

Nach Ausführungsformen ist das Computerprogrammprodukt dazu konfiguriert eine oder mehrere der zuvor genannten Ausführungsformen des Verfahrens zum Modellieren eines patientenindividuellen Zahnersatzteils auszuführen.

Die Erfindung umfasst ferner ein Computersystem zum Modellieren eines patientenindividuellen Zahnersatzteils, wobei das Computersystem ein Speichermedium, einen Prozessor, eine Eingabevorrichtung und eine Anzeigevorrichtung umfasst, wobei auf dem Speichermedium computerlesbare Programminstruktionen zum Modellieren des patientenindividuellen Zahnersatzteils gespeichert sind, wobei ein Ausführen der Programminstruktionen durch den Prozessor des Computersystems das Computersystem dazu veranlasst ein Verfahren zum Modellieren des patientenindividuellen Zahnersatzteils auszuführen, welches umfasst:
- Bereitstellen eines digitalen dreidimensionalen Patientensituationsmodells, wobei das Patientensituationsmodell patientenspezifische Begrenzungsoberflächen von ein oder mehreren Objekten eines Patientengebisses definiert, welche eine Patientensituationsgeometrie definieren, an welche das Zahnersatzteil im Zuge der Modellierung anzupassen ist,
- Bereitstellen eines digitalen dreidimensionalen Zahnersatzteilmodells in einem Ausgangszustand, wobei das Zahnersatzteilmodell zahnersatzteilspezifische Begrenzungsoberflächen des Zahnersatzteils definiert, welche eine Zahnersatzteilgeometrie definieren,
   wobei das Zahnersatzteilmodell in dem Ausgangszustand eine Zahnersatzteilgeometrie in Form einer Ausgangsgeometrie aufweist,
- Bereitstellen ein oder mehrere unter Verwendung der patientenspezifischen Begrenzungsoberflächen definierten geometrischen Anpassungskriterien, welches durch die zahnersatzteilspezifische Begrenzungsoberflächen im Zuge eines patientenindividuellen Anpassens der Zahnersatzteilgeometrie an die Patientensituationsgeometrie einzuhalten sind,
- patientenindividuelles Anpassen der Zahnersatzteilgeometrie des Zahnersatzteilmodells an die Patientensituationsgeometrie des Patientensituationsmodells,
   wobei das patientenindividuelle Anpassen ein Anordnen des Zahnersatzteilmodells in einer von dem Patientensituationsmodell für das Zahnersatzteil bereitgestellten Ausgangsposition umfasst,
   wobei das patientenindividuelle Anpassen ferner ein wiederholtes interaktives Ausführen benutzerdefinierter Veränderungen an dem angeordneten Zahnersatzteilmodell umfasst, wobei das Zahnersatzteilmodell im Zuge jeder der benutzerdefinierten Veränderungen jeweils dynamisch eine Sequenz von Zwischenzuständen durchläuft bis ein aus der jeweiligen benutzerdefinierten Veränderung resultierender Änderungszustand erreicht wird, wobei für jeden der entsprechenden Zwischenzustände sowie den resultierenden Änderungszustand jeweils automatisch aus der Ausgangsgeometrie des Zahnersatzteilmodells eine zustandsspezifische Zustandsgeometrie des Zahnersatzteilmodells unter Einhaltung der geometrischen Anpassungskriterien berechnet wird,
   wobei jede der benutzerdefinierten Veränderungen mittels einer grafischen Benutzeroberfläche auf der Anzeigevorrichtung angezeigt wird, wobei das Anzeigen der jeweiligen benutzerdefinierten Veränderung jeweils ein Anzeigen des dynamischen Durchlaufens der jeweiligen Sequenz von Zwischenzuständen bis zum Erreichen des jeweiligen Änderungszustands durch das Zahnersatzteilmodell mit den jeweiligen hierfür berechneten zustandsspezifische Zustandsgeometrien umfasst,
- Festlegen einer aus dem patientenindividuellen Anpassen des Zahnersatzteilmodells resultierenden Änderungsgeometrie als zum Herstellen des patientenindividuellen Zahnersatzteils zu verwendende patientenindividuellen Zahnersatzteilgeometrie.

Nach Ausführungsformen ist das Computersystem dazu konfiguriert eine oder mehrere der zuvor genannten Ausführungsformen des Verfahrens zum Modellieren eines patientenindividuellen Zahnersatzteils auszuführen.

Die Erfindung umfasst ferner ein Bearbeitungssystem zum Herstellen eines patientenindividuellen Zahnersatzteils, wobei das Bearbeitungssystem ein Computersystem nach einer der zuvor genannten Ausführungsformen umfasst sowie eine Bearbeitungsvorrichtung zum Herstellen des patientenindividuellen Zahnersatzteils aus Zahnersatzmaterial unter Verwendung der patientenindividuellen Zahnersatzteilgeometrie.

Im Weiteren werden Ausführungsformen der Erfindung mit Bezugnahme auf die Zeichnungen näher erläutert. Es zeigen:
- Figur 1: ein schematisches Blockdiagramm eines exemplarischen Computersystems zum Modellieren eines patientenindividuellen Zahnersatzteils,
- Figur 2: ein schematisches Flussdiagramm eines exemplarischen Verfahrens zum Modellieren eines patientenindividuellen Zahnersatzteils,
- Figur 3: ein schematisches Blockdiagramm eines exemplarischen Bearbeitungssystems zum Modellieren eines patientenindividuellen Zahnersatzteils,
- Figur 4: ein schematisches Blockdiagramm eines exemplarischen Bearbeitungssystems zum Modellieren eines patientenindividuellen Zahnersatzteils,
- Figuren 5 A-C: exemplarische Zahnersatzteilmodelle und Patientensituationsmodelle,
- Figuren 6 A-C: exemplarische Anpassungen eines Zahnersatzteilmodells,
- Figuren 7 A-E: eine exemplarische Anpassung eines Zahnersatzteilmodells,
- Figuren 8 A-E: eine exemplarische Anpassung eines Zahnersatzteilmodells,
- Figuren 9A-D: eine exemplarische Anpassung eines Zahnersatzteilmodells,
- Figuren 10A-D: eine exemplarische Anpassung eines Zahnersatzteilmodells,
- Figuren 11 A-C: exemplarische Anpassungen von Zahnersatzteilmodelle und Patientensituationsmodelle,
- Figur 12: eine exemplarische Durchdringungssituation von Zahnersatzteilmodelle und Patientensituationsmodelle,
- Figuren 13 A-C: eine exemplarische Quasi-Abrasion von Zahnersatzteilmodellen,
- Figuren 14 A-C: eine exemplarische Quasi-Abrasion von Zahnersatzteilmodellen,
- Figuren 15 A-C: eine exemplarische Quasi-Abrasion eines Zahnersatzteilmodells,
- Figuren 16 A-C: eine exemplarische Quasi-Abrasion eines Zahnersatzteilmodells
- Figuren 17A-C: eine exemplarische Quasi-Abrasion von Zahnersatzteilmodellen und
- Figuren 18A-C: eine exemplarische Quasi-Abrasion von Zahnersatzteilmodellen.

Elemente der nachfolgenden Ausführungsformen, die einander entsprechen, werden mit denselben Bezugszeichen gekennzeichnet.

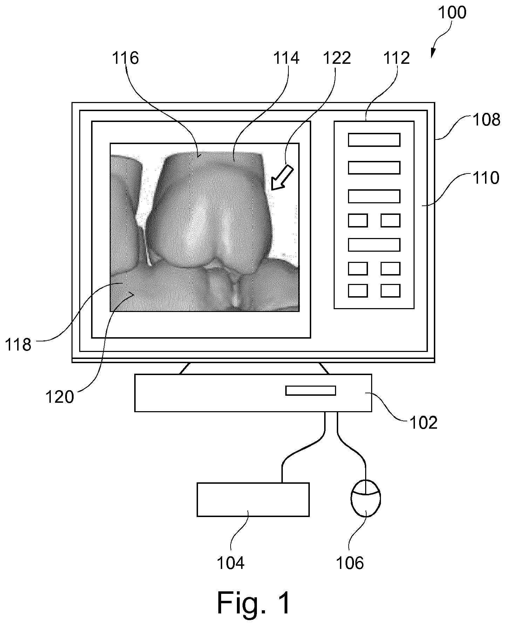

Figur 1 zeigt ein schematisches Blockdiagramm eines Computersystems 100 zum Modellieren eines patientenindividuellen Zahnersatzteils. Das Computersystem 100 umfasst eine Hardwarekomponente 102 mit ein oder mehreren Prozessoren sowie ein oder mehreren Speichermedien. Auf einem oder mehreren der Speichermedien sind computerlesbare Programminstruktionen zum Modellieren des patientenindividuellen Zahnersatzteils gespeichert. Ein Ausführen der Programminstruktionen durch einen oder mehreren der Prozessoren der Hardwarekomponente102 veranlasst das Computersystem 100 ein Verfahren zum Modellieren des patientenindividuellen Zahnersatzteils auszuführen. Das Computersystem 100 umfasst ferner eine Anzeigevorrichtung 108 zum Anzeigen einer grafischen Benutzeroberfläche 110. Das Computersystem 100 umfasst ferner Eingabevorrichtungen, wie beispielsweise eine Tastatur 104 und eine Maus 106, zum Ausführen einer interaktiven Benutzereingabe. Die grafische Benutzeroberfläche 110 umfasst Bedienelemente 112, welche unter Verwendung der Eingabevorrichtungen 104, 106 dazu verwendet werden können, Einstellungen für ein Modellieren eines digitalen dreidimensionalen Zahnersatzteilmodells 114 auszuwählen. Auf der grafischen Benutzeroberfläche 110 wird ferner ein digitales dreidimensionales Zahnersatzteilmodell 114 dargestellt, welches der Nutzer unter Verwendung der Eingabevorrichtungen 104, 106 patientenindividuell an eine ebenfalls auf der grafischen Benutzeroberfläche bereitgestellten Patientensituationsmodell 118 anpassen kann. Das Zahnersatzteilmodell 114 wird durch zahnersatzteilspezifische Begrenzungsoberflächen 116 definiert, welche eine Zahnersatzteilgeometrie festlegen. Das Patientensituationsmodell 118 definiert mit seinen patientenspezifischen Begrenzungsoberflächen 120 ein Patientensituationsgeometrie definiert. Bei den patientenspezifischen Begrenzungsoberflächen 120 des Patientensituationsmodells handelt es sich um Begrenzungsoberflächen von einem oder mehreren Objekten eines Patientengebisses. Ferner umfasst die grafische Benutzeroberfläche beispielsweise ein digitales Bearbeitungswerkzeug 122, welches es dem Benutzer ermöglicht, unter Verwendung der Eingabevorrichtungen 104, 106 das patientenindividuell anzupassende Zahnersatzteilmodell 114 und/oder Teilbereiche des Zahnersatzteilmodells 114 zu selektieren und zu bearbeiten bzw. benutzerdefiniert zu verändern. Entsprechende benutzerdefinierte Veränderungen umfassen beispielsweise ein Verschieben, Rotieren und/oder Skalieren des Zahnersatzteilmodells 114. Die computerlesbaren Programminstruktionen legen ferner geometrische Anpassungskriterien für das benutzerdefinierte Verändern des Zahnersatzteilmodells 114 fest, welche einzuhalten sind. Mit anderen Worten sind lediglich solche Veränderungen zulässig, welche die vordefinierten geometrischen Anpassungskriterien erfüllen. Wird das Zahnersatzteilmodell durch den Nutzer unter Verwendung der Eingabevorrichtungen 104, 106 verändert, beispielsweise relativ zu dem Patientensituationsmodell verschoben, so wird die entsprechende Änderung auf der Benutzeroberfläche 110 als eine dynamische Sequenz von Zwischenzuständen des Zahnersatzteilmodells 114 dargestellt, welche dieses durchläuft bis ein der eingegebenen benutzerdefinierten Veränderungen entsprechender Änderungszustand erreicht ist. Mit anderen Worten kann der Nutzer das Zahnersatzteilmodell beispielsweise innerhalb der grafischen Benutzeroberfläche 110 relativ zu dem Patientensituationsmodell 120 verschieben, drehen und/oder skalieren, wobei die entsprechenden Veränderungen in Echtzeit als dynamische Bildsequenzen dargestellt werden.

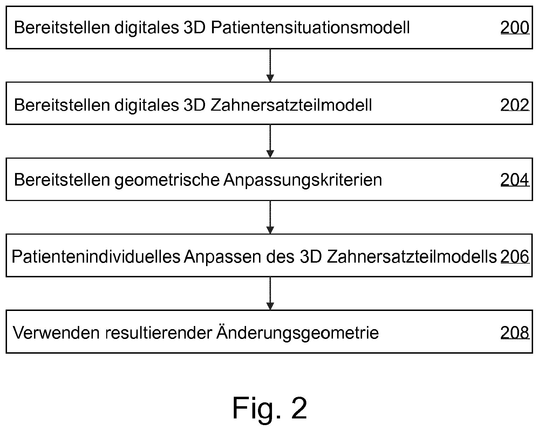

Figur 2 zeigt ein schematisches Ablaufdiagramms eines exemplarischen Verfahrens zum Modellieren eines patientenindividuellen Zahnersatzteils. In Block 200 wird ein digitales dreidimensionales Patientensituationsmodell bereitgestellt. Das Patientensituationsmodell definiert patientenspezifische Begrenzungsoberflächen von ein oder mehreren Objekten eines Patientengebisses. Mit anderen Worten gibt das Patientensituationsmodell eine Ausgangssituation im Gebiss eines Pateienten wieder, an welches das Zahnersatzteil im Zuge der Modellierung anzupassen ist. Die patientenspezifische Begrenzungsoberflächen definieren eine Patientensituationsgeometrie bzw. Flächenstruktur. Das Patientensituationsmodell bzw. die Begrenzungsoberflächen werden beispielsweise mittels einer polygonalen Netzstruktur, z.B. mittels Dreiecke, mittels einer Punktwolke, mittels einer 3D-Volumendatenstruktur oder mittels eines 3D-Signed-Distance-Fields beschrieben. Das Pateientensituationsmodell wird beispielsweise durch eine Vermessung des Patientengebisses bzw. von Objekten des Patientengebisses direkt im Mund des Patienten oder indirekt durch Vermessung mindestens eines Abdrucks oder Modells des Patientengebisses bzw. von Objekten des Patientengebisses, z.B. aus Gips oder Kunststoff, erzeugt. Bei der Vermessung können beispielswiese Röntgenaufnahmen, tomosynthetische Aufnahmen und/oder computertomographische Aufnahme zum Einsatz kommen. Ferner kann das Patientensituationsmodell ein oder mehrere bereits modellierte digitale dreidimensionale Zahnersatzteilmodelle umfassen, d.h. Objekte, welche für einen Einsatz im bzw. Am Patientengebiss bereits fest vorgehenden sind und bei der Anpassung des Zahnersatzteil im Zuge der Modellierung ebenfalls zu berücksichtigen sind.

In Block 202 wird ein digitales dreidimensionales Zahnersatzteilmodell in einem Ausgangszustand bereitgestellt. Wie das Patientensituationsmodell ist auch das Zahnersatzteilmodell über Begrenzungsoberflächen, d.h. zahnersatzteilspezifische Begrenzungsoberflächen, definiert. Diese Begrenzungsoberflächen beschreiben eine Zahnersatzteilgeometrie. Das Zahnersatzteilmodell bzw. die Begrenzungsoberflächen werden beispielsweise mittels einer polygonalen Netzstruktur, etwa mittels Dreiecke, mittels einer Punktwolke, mittels einer 3D-Volumendatenstruktur oder mittels eines 3D-Signed-Distance-Fields beschrieben. Bei dem Zahnersatzteilmodell kann es sich beispielsweise um ein generisches Modell, etwa eines Zahns, handeln, welches aus einer Bibliothek geladen wird, um eine Kopie eines von dem Patientensituationsmodell umfassten Objekts, um eine Kopie eines bereits angepassten patientenindividuellen Zahnersatzteilmodells, oder um ein teilweise angepasstes patientenindividuellen Zahnersatzteilmodell. Das Zahnersatzteilmodell weist in dem Ausgangszustand eine Zahnersatzteilgeometrie in Form einer Ausgangsgeometrie auf.

In Block 204 werden ein oder mehrere unter Verwendung der patientenspezifischen Begrenzungsoberflächen definierte geometrischen Anpassungskriterien festgelegt. Diese Anpassungskriterien sind durch die zahnersatzteilspezifische Begrenzungsoberflächen im Zuge eines patientenindividuellen Anpassens der Zahnersatzteilgeometrie an die Patientensituationsgeometrie einzuhalten. Mit anderen Worten werden nur Anpassungen der Zahnersatzteilgeometrie ermöglicht, welche die Anpassungskriterien erfüllen bzw. werden benutzerdefinierter Veränderungen zum Anpassen der Zahnersatzteilgeometrie so umgesetzt, dass diese die Anpassungskriterien erfüllen. Die Anpassungskriterien definieren beispielsweise einen zulässigen maximalen und/oder minimalen positiven und/oder negativen Abstand bzw. Offset zwischen einer patientenspezifische Begrenzungsoberflächen und einer zahnersatzteilspezifische Begrenzungsoberflächen. Ferner definieren die Anpassungskriterien beispielsweise eine zulässigen Materialmindeststärke, d.h. einen zulässigen minimalen Offset zwischen zwei zahnersatzteilspezifische Begrenzungsoberflächen. Führt eine Benutzer definierte Veränderung, etwa ein Verschieben des Zahnersatzteilmodells relativ zum Patientensituationsmodell, dazu dass ein Anpassungskriterium verletzt werden würde, etwa indem eine zahnersatzteilspezifische Begrenzungsoberflächen eine patientenspezifische Begrenzungsoberflächen durchdringen und ein zulässiger maximaler negativer Offset überschritten werden würde, so wird die Veränderung nur in dem Umfang umgesetzt, wie sie mit dem Anpassungskriterium vereinbar ist. Im Falle der Verschiebung führt dies beispielsweise dazu, dass sich solche zahnersatzteilspezifische Begrenzungsoberflächen, welche einen gemäß den Anpassungskriterien zulässigen maximalen Verschiebungszustand relativ zu einer patientenspezifische Begrenzungsoberfläche erreicht haben, nicht mehr weiter gegenüber der entsprechenden patientenspezifische Begrenzungsoberfläche verschieben lassen. Zahnersatzteilspezifische Begrenzungsoberflächen, welche in keinem Konflikt mit einem der Anpassungskriterien stehen, z.B. noch keinen zulässigen maximalen Verschiebungszustand erreicht haben, lassen sich weiter verschieben, was zu einer Verformung der Zahnersatzteilgeometrie führt. Hierbei erfolgt die Verformung beispielsweise dergestalt, dass geometrische Grundbeziehungen der Zahnersatzteilgeometrie, d.h. charakteristische Eigenschaften der Form des Zahnersatzteils, so weit als möglich beibehalten werden. Geometrische Grundbeziehungen der Zahnersatzteilgeometrie können beispielsweise Abstandverhältnisse, Krümmungsverhältnisse etc. umfassen. Die Anpassungskriterien können beispielsweise vordefiniert sein und/oder durch den Nutzer eingestellt werden. Eine Verformung der Zahnersatzteilgeometrie kann beispielsweise unter Verwendung eines Laplace-Deformationsverfahrens berechnet werden.

In Block 206 erfolgt ein patientenindividuelles Anpassen der Zahnersatzteilgeometrie des Zahnersatzteilmodells an die Patientensituationsgeometrie des Patientensituationsmodells. Hierzu wird das Zahnersatzteilmodell in einer von dem Patientensituationsmodell für das Zahnersatzteil bereitgestellten Ausgangsposition angeordneten. Ferner werden wiederholt interaktive benutzerdefinierter Veränderungen an dem angeordneten Zahnersatzteilmodell ausgeführt. Diese Veränderungen umfassen beispielsweise ein Skalieren einer Erstreckung des Zahnersatzteilmodells entlang einer vordefinierten Erstreckungsrichtung des Zahnersatzteilmodells, ein Verschieben des Zahnersatzteilmodells relativ zu dem Patientensituationsmodell und/oder ein Rotieren des Zahnersatzteilmodells relativ zu dem Patientensituationsmodell. Beispielsweise wird mindestens ein Teilbereich einer Begrenzungsoberfläche des auf der graphischen Benutzeroberfläche visuell wiedergegebenen Zahnersatzteilmodells mittels eines von der graphischen Benutzeroberfläche bereitgestellten interaktiven digitalen Bearbeitungswerkzeugs selektiert und interaktiv bearbeitet.

Dabei durchläuft das Zahnersatzteilmodell im Zuge jeder der benutzerdefinierten Veränderungen jeweils dynamisch eine Sequenz von Zwischenzuständen bis ein aus der jeweiligen benutzerdefinierten Veränderung resultierender Änderungszustand erreicht wird. Für jeden der entsprechenden Zwischenzustände sowie den resultierenden Änderungszustand wird jeweils automatisch aus der Ausgangsgeometrie des ersten Zahnersatzteilmodells eine zustandsspezifische Zustandsgeometrie des Zahnersatzteilmodells in dem entsprechenden Zwischenzustand oder Änderungszustand unter Einhaltung der geometrischen Anpassungskriterien berechnet. Jeder der benutzerdefinierten Veränderungen mittels einer grafischen Benutzeroberfläche auf einer Anzeigevorrichtung angezeigt wird. Dabei erfolgt die Anzeige der benutzerdefinierten Veränderungen beispielsweise jeweils simultan zu ihrer Eingabe. Das Anzeigen der jeweiligen benutzerdefinierten Veränderung umfasst jeweils ein Anzeigen des dynamischen Durchlaufens der jeweiligen Sequenz von Zwischenzuständen bis zum Erreichen des jeweiligen Änderungszustands durch das erste Zahnersatzteilmodell mit den jeweiligen hierfür berechneten zustandsspezifische Zustandsgeometrien. Dabei kann die Anzahl und Schrittweite der von der dynamischen Sequenz umfassten Zwischenzustände vordefiniert und/oder durch den Nutzer eingestellt werden. Nach Ausführungsformen kann sie automatisch in Abhängigkeit von der zur Verfügung stehenden Rechenleistung des Computersystems angepasst werden.

In Block 208 wird eine aus dem patientenindividuellen Anpassen des ersten Zahnersatzteilmodells resultierenden Änderungsgeometrie dazu verwendet eine patientenindividuelle Zahnersatzteilgeometrie für das Herstellen des patientenindividuellen Zahnersatzteils bereitzustellen. Beispielsweise wird die resultierenden Änderungsgeometrie als patientenindividuelle Zahnersatzteilgeometrie für das herzustellende Zahnersatzteil verwendet. Hierzu wird, etwa auf einen Ausgabebefehl des Nutzers zur Ausgabe des konstruierten Zahnersatzteils hin ein digitaler Datensatzes mit patientenindividuelle Zahnersatzteilgeometrie zum automatisierten Herstellen des physischen Zahnersatzteil, eines Zahnersatzteil-Halbzeugs oder eines Prototyps des Zahnersatzteil aus Zahnersatzmaterial, z.B. Zahnrestaurationsmaterial, erzeugt. Die automatisierte Herstellung erfolgt beispielsweise mittels CAM- oder Rapid-Prototyping-Verfahren, wie z.B. CNC-Fräsen oder 3D-Druck. Ein Zahnersatzteil-Halbzeug ist ein Halbzeug, welches eine zahnersatzähnliche Form aufweist und aus welchem durch weitere nachfolgende, z.B., manuelle Bearbeitungsschritte das Zahnersatzteil hergestellt wird.

Nach alternativen Ausführungsformen umfasst das Bereitstellen der resultierenden Änderungsgeometrie ein Übertragen dieser resultierenden Änderungsgeometrie auf ein zweites digitales dreidimensionales Zahnersatzteilmodells desselben Zahnersatzteils. Diese zweite Zahnersatzteilmodell weist eine höhere Auflösung als das erste Zahnersatzteilmodell auf. Führ dieses zweite Zahnersatzteilmodell kann sodann das zuvor beschrieben Verfahren wiederholt werde, wobei der Ausgangzustand des zweiten Zahnersatzteilmodells entweder durch einen generischen Ausgangszustand, d.h. unabhängig von der übernommenen Änderungsgeometrie, oder durch die übernommene Änderungsgeometrie definiert wird. Unabhängig von der übernommenen Änderungsgeometrie bedeutet hier, dass die übernommene Änderungsgeometrie als eine erste benutzerdefinierte Veränderung behandelt wird.

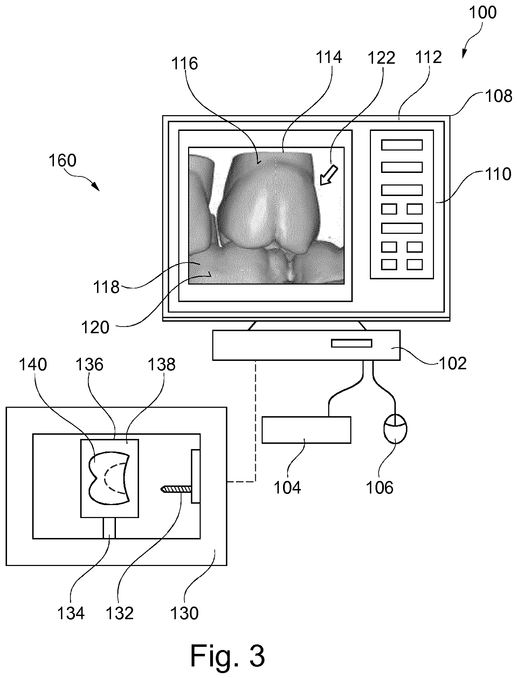

Figur 3 zeigt ein schematisches Blockdiagramm eines Bearbeitungssystems 160 zum Herstellen eines patientenindividuellen Zahnersatzteils. Das Bearbeitungssystem 160 umfasst ein Computersystem 100 zum Modellieren eines patientenindividuellen Zahnersatzteils gemäß Figur 1. Die computerlesbaren Programminstruktionen des Computersystems 100 sind ferner dazu konfiguriert, eine Bearbeitungsvorrichtung 130 zum Herstellen des patientenindividuellen Zahnersatzteils 140 aus Zahnersatzmaterial bzw. Zahnrestaurationsmaterial 138 eines Rohlings 136 herzustellen. Für die Herstellung wird beispielsweise eine patientenindividuelle Zahnersatzteilgeometrie verwendet, welche das Resultat des Modellierens des patientenindividuellen Zahnersatzteils unter Verwendung des Computers 100 ist. Die entsprechende Zahnersatzteilgeometrie wird beispielsweise als Serienmodell bereitgestellt und das Computersystem 100 steuert die Bearbeitungsvorrichtung 130, bei welcher es sich beispielsweise um eine CAM-Bearbeitungsvorrichtung handelt, gemäß der patientenindividuellen Zahnersatzteilgeometrie. Dabei wird die Bearbeitungsvorrichtung 130 beispielsweise so angesteuert, dass mit einem Bearbeitungswerkzeug 132 unter Verwendung eines spanabhebenden Bearbeitungsverfahrens aus dem Rohling 136 ein Zahnersatzteil 140 herausgearbeitet wird, dessen Geometrie der patientenindividuellen Zahnersatzteilgeometrie entspricht. Hierzu stellt die Bearbeitungsvorrichtung 130 den Rohling 136 bereit, welcher von einer Haltevorrichtung 134 gehalten wird.

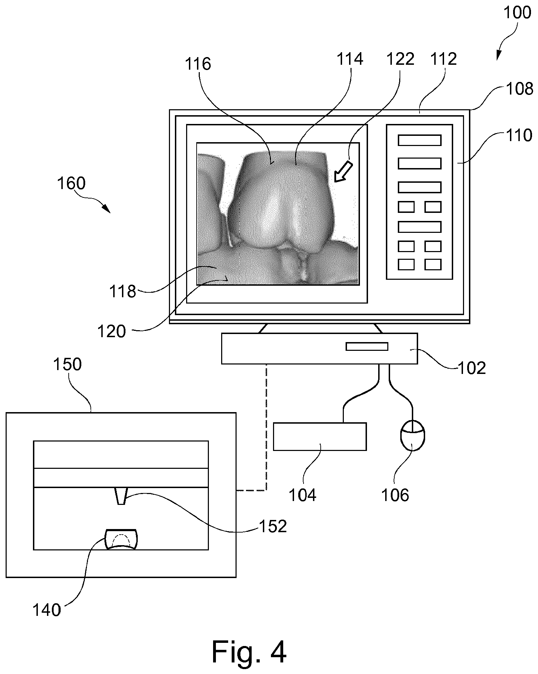

Figur 4 zeigt ein schematisches Blockdiagramm eines alternativen Bearbeitungssystems 160, welches neben einem Computersystem 100, das dem Computersystem 100 aus Figur 1 entspricht, einen 3D-Drucker 150 als eine Bearbeitungsvorrichtung zum Herstellen des patientenindividuellen Zahnersatzteils 140 aus Zahnersatzmaterial unter Verwendung der von dem Computersystem 100 bereitgestellten patientenindividuellen Zahnersatzteilgeometrie ermöglicht. Der 3D-Drucker 150 umfasst ein Druckelement 152, mit welchem das Zahnersatzmaterial schichtweise ausgegeben wird, sodass schichtweise das patientenindividuelle Zahnersatzteil 140 gemäß der patientenindividuellen Zahnersatzteilgeometrie erstellt wird.

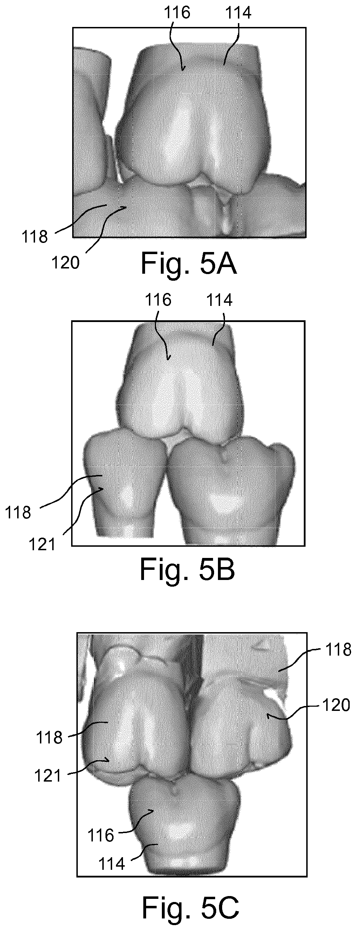

Figuren 5A bis 5C zeigen unterschiedliche Typen von Patientensituationsmodellen 118, welche für das Modellieren des patientenindividuellen Zahnersatzteils verwendet werden. Figur 5A zeigt eine Situation, bei welcher das Patientensituationsmodell 118 auf einem Scan von einem oder mehreren Objekten eines Patientengebisses beruht. Das Patientensituationsmodell 118 wird von den eingescannten patientenspezifischen Begrenzungsoberflächen 120 definiert. Dabei kann es sich um direkt eingescannte Begrenzungsoberflächen 120 der entsprechenden Objekte handeln oder um einen Scan eines Negativabdrucks oder Positivabdrucks der tatsächlichen Objekte des Patientengebisses. An dieses Patientensituationsmodell 118 wird ein digitales dreidimensionales Zahnersatzteilmodell 114 angepasst, welches von Begrenzungsoberflächen 116 definiert wird. Figur 5B zeigt eine Situation, in welcher es sich bei den Objekten des Patientengebisses um bereits modellierte digitale dreidimensionale Zahnersatzteilmodelle mit modellierten Begrenzungsoberflächen 121 handelt, welche das Patientensituationsmodell 118 bilden und an welche das Zahnersatzteilmodell 114 angepasst wird. Figur 5C zeigt schließlich ein Patientensituationsmodell 118, welches definiert wird aus einer Kombination von gescannten patientenspezifischen Begrenzungsoberflächen 120 und modellierten Begrenzungsoberflächen 121 eines für das Patientengebiss modellierten dreidimensionalen Zahnersatzteilmodells. An dieses Patientensituationsmodell wird das digitale dreidimensionale Zahnersatzteilmodell 114 patientenindividuell angepasst.

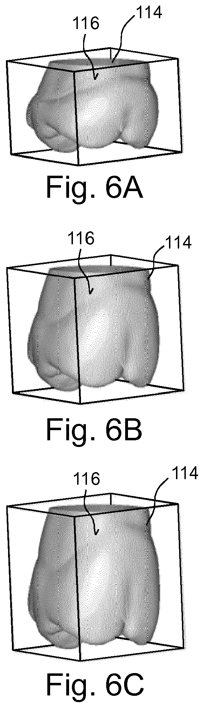

Die Figuren 6A bis 6C zeigen eine beispielhafte benutzerdefinierte Veränderung eines Zahnersatzteilmodells 114, welches zahnersatzteilspezifische Begrenzungsoberflächen 116 definiert. Bei der beispielhaften benutzerdefinierten Veränderung handelt es sich um ein Skalieren, bei welchem eine gegenüberliegende Seite der Zahnersatzteilgeometrie festgehalten wird. Das Skalieren wird beispielsweise entlang einer Hauptachse des Zahns, um welche es sich beispielsweise um eine okklusale Achse, eine mesiale Achse oder eine bukkale Achse handeln kann. Figur 6B zeigt eine Ausgangssituation der Zahnersatzteilgeometrie, Figur 6A zeigt eine entlang der okklusalen Achse reduzierte Skalierung der entsprechenden Zahnersatzteilgeometrien, während Figur 6C eine entlang der okklusalen Achse vergrößerte Skalierung der Zahnersatzteilgeometrien zeigt.

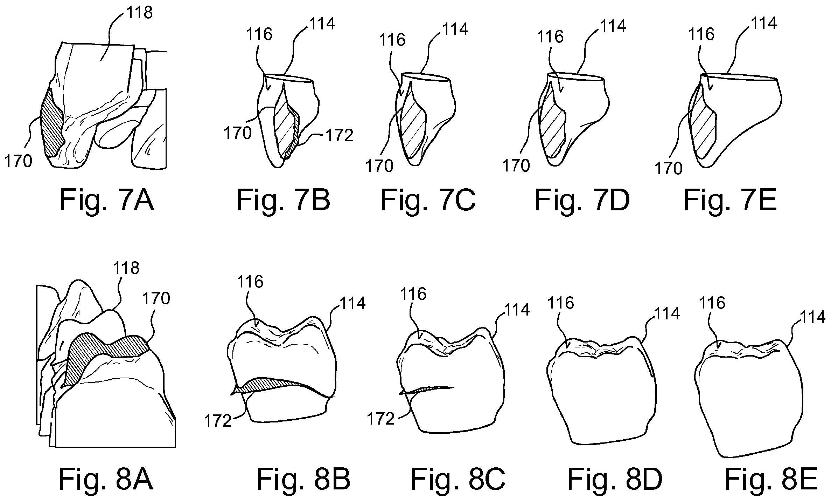

Die Figuren 7A bis 7E zeigen eine schrittweise Deformation eines Zahnersatzteilmodells 114 für einen Frontzahn unter Berücksichtigung eines Anpassungskriteriums in Form einer vorbestimmten Mindestdicke. Die Mindestdicke wird präsentiert durch die Grenzfläche 170, welche die zahnersatzteilspezifische Begrenzungsoberflächen 116, in diesem Fall die bukkale Begrenzungsfläche, nicht überschreiten dürfen. Wird das Zahnersatzteilmodells 114 auf der Grenzfläche 170 angeordnet, kann diese beispielsweise in einem hinteren Bereich, welcher nicht von der bukkalen Begrenzungsfläche umfasst ist, aus dem Zahnersatzteilmodell 114 herausragen. So durch durchdring der Bereich 172 der Grenzfläche 170 das Zahnersatzteilmodell 114. Wird der Frontzahn bzw. das Zahnersatzteilmodell 114 des Frontzahns in lingualer Richtung verschoben, so führt dies zu einer Verformung des Frontzahns, bei welcher der Durchmesser in lingualer Richtung zunimmt, da die bukkale Begrenzungsfläche des Zahnersatzteilmodells 114 die vorgegebene Grenzfläche 170 nicht überschreiten kann. Hierbei wird die Grenzfläche 170 zusehends komplett umschlossen. In den Figuren 8A bis 8E ist eine entsprechende Situation für einen Backenzahn, d. h. prämolar oder molar, dargestellt. Wiederum wird die Mindestdicke durch eine Grenzfläche 170 definiert, welche in diesem Fall die Form eines Zahns aufweist. Wird das Zahnersatzteilmodell 114 in okklusaler Richtung verschoben, so darf die Okklusalfläche als Begrenzungsoberfläche 116 gemäß dem Anpassungskriterium die vordefinierte Grenzfläche 170 nicht durchdringen, wodurch es zu einer Abflachung der Höcker und einer Streckung des Zahnersatzteilmodells 114 in okklusaler Richtung kommt. Zunächst kann ein Bereich 172 der Grenzfläche 170 das Zahnersatzteilmodell 114 unterhalb der Okklusalfläche durchdringen. Wird aber das Zahnersatzteilmodells 114 in okklusaler Richtung soweit nach unten verschoben, dass die Okklusalfläche in den Bereich der Grenzfläche 170 kommt, so wird die Okklusalfläche über die Grenzfläche 170 geschoben und die Durchdringungen 172 verschwinden. Die Figuren 7B bis 7E und 8B bis 8E illustrieren zudem jeweils eine dynamische Sequenz, welche im Zuge einer benutzerdefinierten Veränderung zu der in Figur 7E bzw. Figur 8E jeweils gezeigten Änderungsgeometrie des Zahnersatzteilmodells 114.

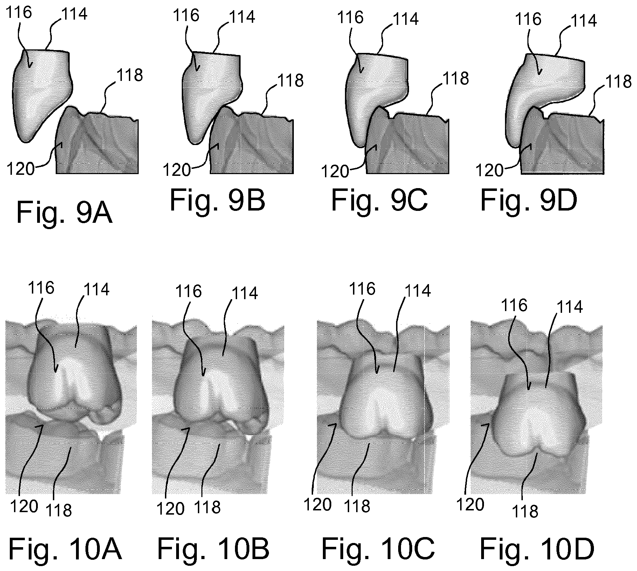

Die Figuren 9A bis 9D zeigen eine Anpassung eines Zahnersatzteilmodells 114 für einen Frontzahn an einen von einem Patientensituationsmodell 118 umfassten Antagonisten unter Berücksichtigung eines Anpassungskriteriums, gemäß welchem keine Durchdringung zwischen dem anzupassenden Zahnersatzteilmodell 114 und dem Antagonisten des Patientensituationsmodells 118 auftreten darf. Hierbei kommt es wie bereits im Fall der Figuren 7A bis 7E zu einer Erhöhung des Durchmessers des Frontzahns in lingualer Richtung, wobei zugleich die bukkale Begrenzungsfläche des Zahnersatzteilmodells 114 nahezu unverändert beibehalten wird. Die Figuren 9A bis 9B illustrieren eine dynamische Sequenz, welche im Zuge einer benutzerdefinierten Veränderung zu der in Figur 9E gezeigten Änderungsgeometrie des Zahnersatzteilmodells 114.

Die Figuren 10A bis 10D zeigen eine Anpassung eines Zahnersatzteilmodells 114 für einen Backenzahn unter demselben Anpassungskriterium wie bereits in den Figuren 9A bis 9D, gemäß dem eine Durchdringung des Zahnersatzteilmodells 114 und eines Antagonisten eines Patientensituationsmodells 118 untersagt ist. In diesem Fall erfolgt eine teilweise Streckung der okklusalen Fläche in okklusaler Richtung, wobei sich das Zahnersatzteilmodell 114 um den Antagonisten herumerstreckt. Die Figuren 9A bis 9B illustrieren eine dynamische Sequenz, welche im Zuge einer benutzerdefinierten Veränderung zu der in Figur 9E gezeigten Änderungsgeometrie des Zahnersatzteilmodells 114.

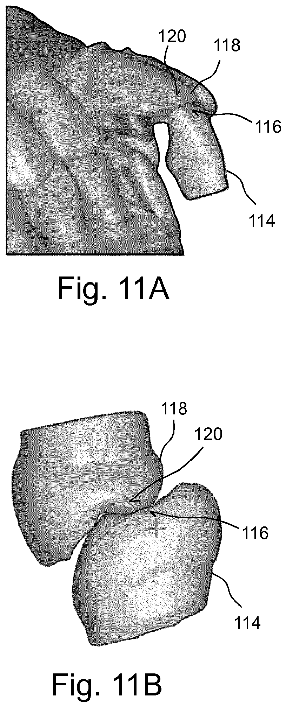

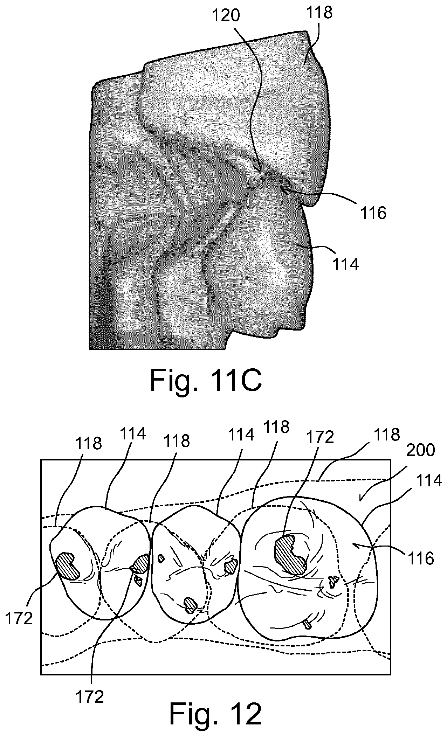

Figur 11A zeigt eine Anpassung eines Zahnersatzteilmodells 114, bei welchem das entsprechende Zahnersatzteilmodell 114 unverformt bleibt und ein Anpassungskriterium, beispielsweise ein Ausschluss einer Durchdringung mit einem Antagonisten eines Patientensituationsmodells 118, durch den entsprechenden Antagonisten aufgefangen wird. Hier wird der entsprechend Antagonist aus seiner Ursprungsposition rotiert. Figur 11B zeigt ein Beispiel, bei welchem das Anpassungskriterium sowohl durch das Zahnersatzteilmodell 114 als auch durch einen Antagonisten des Patientensituationsmodells 118 erfüllt wird, indem beide verformt werden. Figur 11C zeigt ein Beispiel, bei welchem das Anpassungskriterium ausschließlich durch eine Deformation des anzupassenden Zahnersatzteilmodells 114 erfüllt wird, während das Patientensituationsmodell 118 unverändert bleibt.

Figur 12 zeigt Durchdringungen 172 einer okklusalen Begrenzungsoberfläche 116 eines Zahnersatzteilmodells 114 mit einer okklusalen Begrenzungsfläche 120 eines Patientensituationsmodells 118, in diesem Fall eines Antagonisten. Die entsprechenden Bereiche, in denen eine Durchdringung 172 vorliegt, können beispielsweise durch ein Abschneiden der entsprechenden Bereiche 172 des Zahnersatzteilmodells 114 oder durch eine lokale Deformation der okklusalen Begrenzungsfläche 116 des Zahnersatzteilmodells 114 in diesen Bereichen 172 gelöst werden. Die Figur 12 zeigt Durchdringungen 172 für eine relative Positionierung von Zahnersatzteilmodells 114 und Patientensituationsmodells 118. Im Falle eines virtuellen Artikulators werden beispielsweise entsprechende Darstellungen für eine Mehrzahl unterschiedlicher Positionierungen von Zahnersatzteilmodells 114 und Patientensituationsmodells 118 zusammen mit gegebenenfalls auftretenden Durchdringungen 172 gezeigt. Die unterschiedlichen Positionierungen entsprechen dabei unterschiedlichen relativen Positionierungen, welche im Zuge einer Kaubewegung durchlaufen werden. Die gegebenenfalls Durchdringungen 172 können nacheinander in Form einer dynamischen Sequenz dargestellt werden, bei welcher die unterschiedlichen Positionierungen nacheinander durchlaufen werden. Somit kann der Nutzer nacheinander für jede Positionierung prüfen, ob Anpassungen aufgrund auftretender Durchdringungen 172 notwendig sind und diese gegebenenfalls ausführen. Alternativ kann auf das Zahnersatzteilmodells 114 eine Überlagerung der Durchdringungen 172 für die unterschiedlichen Positionierung projiziert werden. Somit kann der Nutzer auf einen Blick prüfen, ob Anpassungen aufgrund von Durchdringungen 172 notwendig sind und diese gegebenenfalls ausführen. Hierbei kann er beim Anpassen aufgrund der Überlagerung alle Durchdringungen 172 für alle Positionierungen gleichzeitig berücksichtigen.

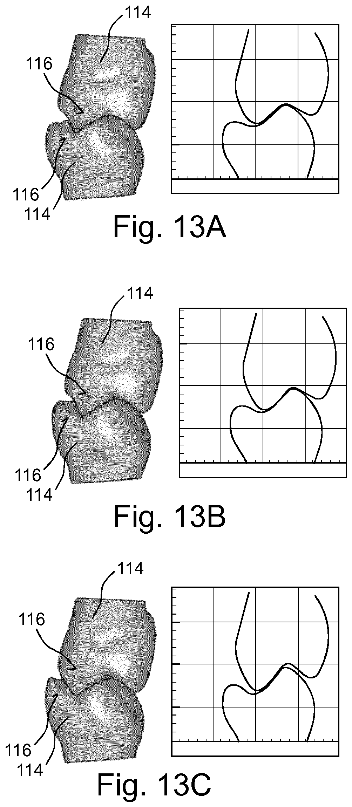

Figuren 13A bis 13C zeigen ein Implementieren einer Quasi-Abrasion, bei welcher die zervikalen Grenzen beibehalten werden und die Okklusion zwischen den durch die beiden Zahnersatzmodelle 116 gebildeten Antagonisten im Wesentlichen beibehalten werden. Der linke Teil der Figuren 13A bis 13C zeigt jeweils die Zahnersatzmodelle 116 mit ihren Begrenzungsoberflächen 118, der rechte Teil zeigt jeweils einen Querschnitt durch die Zahnersatzmodelle 116. Von der Figur 13A über die Figur 13B zur Figur 13C nimmt in dem gezeigten Beispiel die Abrasion ab, d. h. die relativen Höhen der Höcker bzw. Tiefen der Fissuren der Okklusalfläche nehmen zu.

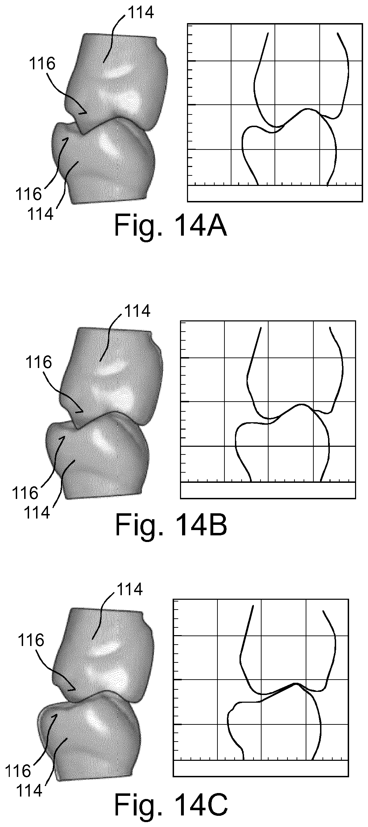

Die Figuren 14A bis 14C zeigen eine Implementierung einer Quasi-Abrasion, bei welcher die Abrasion zunimmt, ausgehend von der Figur 14A, welche identisch ist mit der Figur 13A, über die Figur 14B zur Figur 14C. Im Zuge der Zunahme der Abrasion nimmt die Höhe der Höcker bzw. die Tiefe der Fissuren in der Okklusalfläche ab. Wie zuvor im Falle der Figuren 13A bis 13C werden die zervikalen Grenzen festgehalten und die Okklusion wird nahezu unverändert beibehalten.

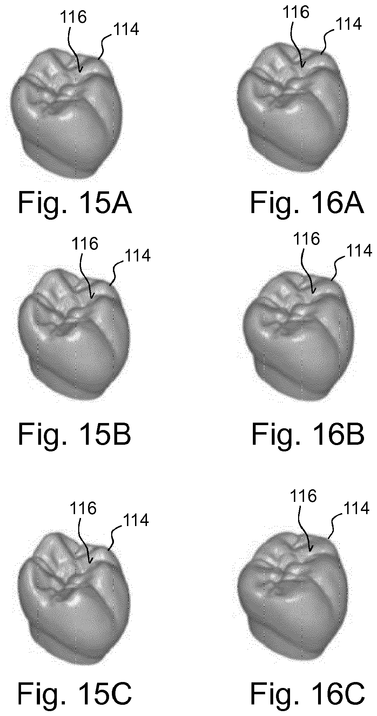

Die Figuren 15A bis 15C zeigen, wie zuvor die Figuren 13A bis 13C, eine Abnahme einer Quasi-Abrasion, welche eher jüngeren Zähnen entspricht, während die Figuren 16A bis 16C eine Zunahme einer Quasi-Abrasion für ein Zahnersatzteilmodell 114 mit seinen Begrenzungsoberflächen 116 zeigen, durch welche ein Alterungsprozess der Zähne nachgestellt werden kann. Die Figuren 16A bis 16C entsprechen dabei den Figuren 14A bis 14C. Im Falle einer solchen virtuellen Alterung des Zahnersatzteilmodells 114 wird die Ausgangsgeometrie algorithmisch so verformt, dass sie bestimmte Eigenschaften erfüllt, die typischerweise bei Zähnen von älteren Patienten zu finden sind. Diese Eigenschaften umfassen beispielsweise flachere Fissuren und/oder stärkere Glättung der anatomischen Strukturen der Zahnoberfläche.

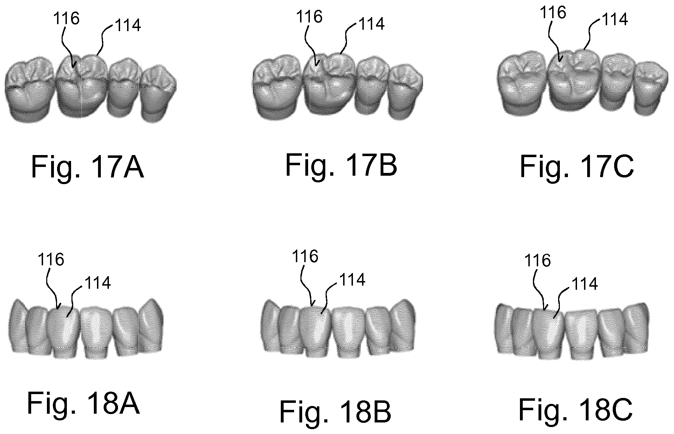

In den Figuren 17A bis 17C ist eine entsprechende Quasi-Abrasion für eine Mehrzahl von Zahnersatzteilmodellen 114 für Backenzähne gezeigt, wobei die Figur 17B eine Ausgangssituation zeigt, relativ zu der die Abrasion in Figur 17A abnimmt, während sie in der Figur 17C relativ zunimmt.

Die Figuren 18A bis 18C zeigen eine entsprechende Quasi-Abrasion für eine Mehrzahl von Zahnersatzteilmodellen 114 für Frontzähne, bei welchen sich ein entsprechender Alterungsprozess insbesondere durch ein Glätten der inzisalen Begrenzungsoberflächen 116 bzw. der von dieser umfassten Inzisalkante zeigt. Wiederum zweigt Figur 18B eine Ausgangssituation, gegenüber der die Quasi-Abrasion in Figur 18A abnimmt, während sie in Figur 18C relativ zunimmt.

### Bezugszeichenliste

- 100: Computersystem
- 102: Hardwarekomponente
- 104: Eingabevorrichtung
- 106: Eingabevorrichtung
- 108: Anzeigevorrichtung
- 110: grafischen Benutzeroberfläche
- 112: Bedienelemente
- 114: Zahnersatzteilmodell
- 116: Begrenzungsoberflächen
- 118: Patientensituationsmodell
- 120: Begrenzungsoberflächen
- 121: Begrenzungsoberflächen
- 122: digitales Bearbeitungswerkzeug
- 130: Bearbeitungsvorrichtung
- 132: Bearbeitungswerkzeug
- 134: Haltevorrichtung
- 136: Rohling
- 138: Zahnersatzmaterial
- 140: Zahnersatzteil
- 150: 3D-Drucker
- 152: Druckelement
- 160: Bearbeitungssystems
- 170: Grenzfläche
- 172: Durchdringungsbereich

## Patentansprüche

1. Computerimplementiertes Verfahren zum Modellieren eines patientenindividuellen Zahnersatzteils (140), wobei das Verfahren umfasst:
• Bereitstellen eines digitalen dreidimensionalen Patientensituationsmodells (118), wobei das Patientensituationsmodell patientenspezifische Begrenzungsoberflächen (120, 121) von ein oder mehreren Objekten eines Patientengebisses definiert, welche eine Patientensituationsgeometrie definieren, an welche das Zahnersatzteil im Zuge der Modellierung anzupassen ist,
• Bereitstellen eines ersten digitalen dreidimensionalen Zahnersatzteilmodells (114) in einem Ausgangszustand, wobei das erste Zahnersatzteilmodell zahnersatzteilspezifische Begrenzungsoberflächen (116) des Zahnersatzteils definiert, welche eine Zahnersatzteilgeometrie definieren,
wobei das erste Zahnersatzteilmodell in dem Ausgangszustand eine Zahnersatzteilgeometrie in Form einer Ausgangsgeometrie aufweist,
• Bereitstellen ein oder mehrere unter Verwendung der patientenspezifischen Begrenzungsoberflächen definierten geometrischen Anpassungskriterien, welche durch die zahnersatzteilspezifische Begrenzungsoberflächen im Zuge eines patientenindividuellen Anpassens der Zahnersatzteilgeometrie an die Patientensituationsgeometrie einzuhalten sind,
• patientenindividuelles Anpassen der Zahnersatzteilgeometrie des ersten Zahnersatzteilmodells an die Patientensituationsgeometrie des Patientensituationsmodells,
wobei das patientenindividuelle Anpassen ein Anordnen des ersten Zahnersatzteilmodells in einer von dem Patientensituationsmodell für das Zahnersatzteil bereitgestellten Ausgangsposition umfasst,
wobei das patientenindividuelle Anpassen ferner ein wiederholtes interaktives Ausführen benutzerdefinierter Veränderungen an dem angeordneten ersten Zahnersatzteilmodell umfasst, wobei das erste Zahnersatzteilmodell im Zuge jeder der benutzerdefinierten Veränderungen jeweils dynamisch eine Sequenz von Zwischenzuständen durchläuft bis ein aus der jeweiligen benutzerdefinierten Veränderung resultierender Änderungszustand erreicht wird, wobei für jeden der entsprechenden Zwischenzustände sowie den resultierenden Änderungszustand jeweils automatisch aus der Ausgangsgeometrie des ersten Zahnersatzteilmodells eine zustandsspezifische Zustandsgeometrie des ersten Zahnersatzteilmodells unter Einhaltung der geometrischen Anpassungskriterien berechnet wird,
wobei jede der benutzerdefinierten Veränderungen mittels einer grafischen Benutzeroberfläche (110) auf einer Anzeigevorrichtung (108) angezeigt wird, wobei das Anzeigen der jeweiligen benutzerdefinierten Veränderung jeweils ein Anzeigen des dynamischen Durchlaufens der jeweiligen Sequenz von Zwischenzuständen bis zum Erreichen des jeweiligen Änderungszustands durch das erste Zahnersatzteilmodell mit den jeweiligen hierfür berechneten zustandsspezifische Zustandsgeometrien umfasst,
• Verwenden einer aus dem patientenindividuellen Anpassen des ersten Zahnersatzteilmodells resultierenden Änderungsgeometrie zum Bereitstellen einer patientenindividuellen Zahnersatzteilgeometrie für das Herstellen des patientenindividuellen Zahnersatzteils.

2. Verfahren nach Anspruch 1, wobei das Bereitstellen der resultierenden Änderungsgeometrie ein Verwenden der resultierenden Änderungsgeometrie als patientenindividuelle Zahnersatzteilgeometrie umfasst.

3. Verfahren nach Anspruch 1, wobei das Bereitstellen der resultierenden Änderungsgeometrie ein Übertragen der resultierenden Änderungsgeometrie auf ein zweites digitales dreidimensionales Zahnersatzteilmodells umfasst, wobei das zweite Zahnersatzteilmodell eine höhere Auflösung als das erste Zahnersatzteilmodell aufweist.

4. Verfahren nach Anspruch 1, wobei die geometrischen Anpassungskriterien ein oder mehrere zulässige Maximal- und/oder Minimalwerte für positive und/oder negative Abstände zwischen patientenspezifische Begrenzungsoberflächen des Patientensituationsmodells und zahnersatzteilspezifische Begrenzungsoberflächen des Zahnersatzteilmodells festlegen.

5. Verfahren nach Anspruch 1, wobei die geometrischen Anpassungskriterien ein oder mehrere zulässige Minimalwerte für positive Abstände zwischen zahnersatzteilspezifische Begrenzungsoberflächen des Zahnersatzteilmodells festlegen.

6. Verfahren nach einem der vorangehenden Ansprüche, wobei die geometrischen Anpassungskriterien hierarchisch gegliedert sind und im Falle nicht miteinander vereinbarer geometrischer Anpassungskriterien einzelne geometrischen Anpassungskriterien gemäß der hierarchischen Gliederung Vorrang gegenüber ein oder mehreren anderen geometrischen Anpassungskriterien gewährt bekommen.

7. Verfahren nach einem der vorangehenden Ansprüche, wobei das Anzeigen der benutzerdefinierten Veränderung jeweils in Echtzeit erfolgt.

8. Verfahren nach einem der vorangehenden Ansprüche, wobei die benutzerdefinierten Veränderungen jeweils zumindest eine der folgenden durch eine interaktive Benutzereingabe definierten Veränderungen umfassen: ein Skalieren einer Erstreckung des Zahnersatzteilmodells entlang einer vordefinierten Erstreckungsrichtung des Zahnersatzteilmodells, ein Verschieben des Zahnersatzteilmodells relativ zu dem Patientensituationsmodell und ein Rotieren des Zahnersatzteilmodells relativ zu dem Patientensituationsmodell.

9. Verfahren nach einem der vorangehenden Ansprüche, wobei die Eingabe der benutzerdefinierten Veränderungen jeweils ein Selektieren und interaktives Bearbeiten mindestens eines Teilbereichs einer Begrenzungsoberfläche des auf der graphischen Benutzeroberfläche visuell wiedergegebenen Zahnersatzteilmodells mittels eines von der graphischen Benutzeroberfläche bereitgestellten interaktiven digitalen Bearbeitungswerkzeugs (122) umfasst.

10. Verfahren nach Anspruch 9, wobei das interaktive Bearbeiten ein Verformen eines Teilbereichs und/oder ein Abschneiden eines von dem Teilbereich begrenzten Volumenabschnitts der Zahnersatzteilgeometrie umfasst.

11. Verfahren nach einem der vorangehenden Ansprüche, wobei das Anordnen des Zahnersatzteilmodells in der Ausgangsposition eine automatische Anpassung des Zahnersatzteilmodells an eine in dem Patientensituationsmodell definierte Präparationsgrenze für das Zahnersatzteil umfasst.

12. Verfahren nach einem der vorangehenden Ansprüche, wobei das Verfahren ferner umfasst:
• Auswählen eines Änderungszustands des ersten Zahnersatzteilmodells,
• Simulieren einer Kaubewegung für den ausgewählten Änderungszustands des ersten Zahnersatzteilmodells, wobei das Simulieren der Kaubewegung eine Berechnung einer dynamisch durchlaufenen Sequenz von relativen Positionen des Zahnersatzteilmodells zu einem von dem Patientensituationsmodell umfasste Antagonisten des Zahnersatzteilmodells umfasst, wobei für jede der relativen Positionen mittels der grafischen Benutzeroberfläche auf der Anzeigevorrichtung jeweils zumindest eine okklusale Begrenzungsfläche des Zahnersatzteilmodell und eine okklusale Begrenzungsfläche des Antagonisten angezeigt wird.

13. Verfahren nach Anspruch 12, wobei für die einzelnen relativen Positionen der dynamischen Sequenz jeweils Teilbereiche der okklusalen Begrenzungsfläche des Zahnersatzteilmodells angezeigt werden, welche die okklusale Begrenzungsfläche des Antagonisten durchdringen.

14. Verfahren nach einem der vorangehenden Ansprüche, wobei es sich bei dem bereitgestellten Zahnersatzteilmodell in dem Ausgangszustand um ein generisches Modell für das Zahnersatzteil handelt.

15. Verfahren nach einem der vorangehenden Ansprüche, wobei die Objekte eines Patientengebisses ein oder mehrere der folgenden Objekte umfassen: einen Zahn, einen Zahnstumpf, Zahnfleisch, einen Zahnersatz, ein Implantat, einen Zahnhalteapparat, einen Locator, eine Aufbissschiene, ein Steg, eine Prothese oder eine Teilprothese, eine Modellgussprothese, ein Zahnersatzprovisorium, Zahnfüllung, Inlay.

16. Verfahren nach einem der vorangehenden Ansprüche, wobei das Verfahren ferner ein Herstellen des patientenindividuellen Zahnersatzteils unter Verwendung der als patientenindividuellen Zahnersatzteilgeometrie festgelegten Änderungsgeometrie umfasst.

17. Verfahren nach einem der vorangehenden Ansprüche, wobei die patientenspezifischen und zahnersatzteilspezifischen Begrenzungsoberflächen unter Verwendung eines der folgend Verfahren implementiert sind: einer polygonalen Netzstruktur, wobei Vertices der entsprechenden Netzstruktur und/oder Punkte innerhalb der Polygone der Netzstruktur die entsprechenden Begrenzungsoberflächen definieren, einer Punktewolke, wobei die Punkte der Punktewolke die entsprechenden Begrenzungsoberflächen definieren, einer 3D-Volumendatenstruktur, welche ein Voxelgitter umfasst, oder eines 3D-Signed-Distance-Fields.

18. Computerprogrammprodukt zum Modellieren eines patientenindividuellen Zahnersatzteils (140), welches ein nichtflüchtiges computerlesbares Speichermedium mit computerlesbaren Programminstruktionen zum Modellieren des patientenindividuellen Zahnersatzteils umfasst, wobei ein Ausführen der Programminstruktionen durch einen Prozessor eines Computersystems (100) das Computersystem dazu veranlasst ein Verfahren zum Modellieren des patientenindividuellen Zahnersatzteils auszuführen, welches umfasst:
• Bereitstellen eines digitalen dreidimensionalen Patientensituationsmodells (118), wobei das Patientensituationsmodell patientenspezifische Begrenzungsoberflächen (120, 121) von ein oder mehreren Objekten eines Patientengebisses definiert, welche eine Patientensituationsgeometrie definieren, an welche das Zahnersatzteil im Zuge der Modellierung anzupassen ist,
• Bereitstellen eines digitalen dreidimensionalen Zahnersatzteilmodells (114) in einem Ausgangszustand, wobei das Zahnersatzteilmodell zahnersatzteilspezifische Begrenzungsoberflächen (116) des Zahnersatzteils definiert, welche eine Zahnersatzteilgeometrie definieren,
wobei das Zahnersatzteilmodell in dem Ausgangszustand eine Zahnersatzteilgeometrie in Form einer Ausgangsgeometrie aufweist,
• Bereitstellen ein oder mehrere unter Verwendung der patientenspezifischen Begrenzungsoberflächen definierten geometrischen Anpassungskriterien, welche durch die zahnersatzteilspezifische Begrenzungsoberflächen im Zuge eines patientenindividuellen Anpassens der Zahnersatzteilgeometrie an die Patientensituationsgeometrie einzuhalten sind,
• patientenindividuelles Anpassen der Zahnersatzteilgeometrie des Zahnersatzteilmodells an die Patientensituationsgeometrie des Patientensituationsmodells, wobei das patientenindividuelle Anpassen ein Anordnen des Zahnersatzteilmodells in einer von dem Patientensituationsmodell für das Zahnersatzteil bereitgestellten Ausgangsposition umfasst,
wobei das patientenindividuelle Anpassen ferner ein wiederholtes interaktives Ausführen benutzerdefinierter Veränderungen an dem angeordneten Zahnersatzteilmodell umfasst, wobei das Zahnersatzteilmodell im Zuge jeder der benutzerdefinierten Veränderungen jeweils dynamisch eine Sequenz von Zwischenzuständen durchläuft bis ein aus der jeweiligen benutzerdefinierten Veränderung resultierender Änderungszustand erreicht wird, wobei für jeden der entsprechenden Zwischenzustände sowie den resultierenden Änderungszustand jeweils automatisch aus der Ausgangsgeometrie des Zahnersatzteilmodells eine zustandsspezifische Zustandsgeometrie des Zahnersatzteilmodells unter Einhaltung der geometrischen Anpassungskriterien berechnet wird,
wobei jede der benutzerdefinierten Veränderungen mittels einer grafischen Benutzeroberfläche (110) auf einer Anzeigevorrichtung (108) angezeigt wird, wobei das Anzeigen der jeweiligen benutzerdefinierten Veränderung jeweils ein Anzeigen des dynamischen Durchlaufens der jeweiligen Sequenz von Zwischenzuständen bis zum Erreichen des jeweiligen Änderungszustands durch das Zahnersatzteilmodell mit den jeweiligen hierfür berechneten zustandsspezifische Zustandsgeometrien umfasst,
• Festlegen einer aus dem patientenindividuellen Anpassen des Zahnersatzteilmodells resultierenden Änderungsgeometrie als zum Herstellen des patientenindividuellen Zahnersatzteils zu verwendende patientenindividuellen Zahnersatzteilgeometrie.

19. Computersystem (100) zum Modellieren eines patientenindividuellen Zahnersatzteils (140), wobei das Computersystem ein Speichermedium, einen Prozessor, eine Eingabevorrichtung (104, 106) und eine Anzeigevorrichtung (108) umfasst, wobei auf dem Speichermedium computerlesbare Programminstruktionen zum Modellieren des patientenindividuellen Zahnersatzteils gespeichert sind, wobei ein Ausführen der Programminstruktionen durch den Prozessor des Computersystems das Computersystem dazu veranlasst ein Verfahren zum Modellieren des patientenindividuellen Zahnersatzteils auszuführen, welches umfasst:
• Bereitstellen eines digitalen dreidimensionalen Patientensituationsmodells (118), wobei das Patientensituationsmodell patientenspezifische Begrenzungsoberflächen (120, 121) von ein oder mehreren Objekten eines Patientengebisses definiert, welche eine Patientensituationsgeometrie definieren, an welche das Zahnersatzteil im Zuge der Modellierung anzupassen ist,
• Bereitstellen eines digitalen dreidimensionalen Zahnersatzteilmodells in einem Ausgangszustand, wobei das Zahnersatzteilmodell (114) zahnersatzteilspezifische Begrenzungsoberflächen (116) des Zahnersatzteils definiert, welche eine Zahnersatzteilgeometrie definieren,
wobei das Zahnersatzteilmodell in dem Ausgangszustand eine Zahnersatzteilgeometrie in Form einer Ausgangsgeometrie aufweist,
• Bereitstellen ein oder mehrere unter Verwendung der patientenspezifischen Begrenzungsoberflächen definierten geometrischen Anpassungskriterien, welche durch die zahnersatzteilspezifische Begrenzungsoberflächen im Zuge eines patientenindividuellen Anpassens der Zahnersatzteilgeometrie an die Patientensituationsgeometrie einzuhalten sind,
• patientenindividuelles Anpassen der Zahnersatzteilgeometrie des Zahnersatzteilmodells an die Patientensituationsgeometrie des Patientensituationsmodells,
wobei das patientenindividuelle Anpassen ein Anordnen des Zahnersatzteilmodells in einer von dem Patientensituationsmodell für das Zahnersatzteil bereitgestellten Ausgangsposition umfasst,
wobei das patientenindividuelle Anpassen ferner ein wiederholtes interaktives Ausführen benutzerdefinierter Veränderungen an dem angeordneten Zahnersatzteilmodell umfasst, wobei das Zahnersatzteilmodell im Zuge jeder der benutzerdefinierten Veränderungen jeweils dynamisch eine Sequenz von Zwischenzuständen durchläuft bis ein aus der jeweiligen benutzerdefinierten Veränderung resultierender Änderungszustand erreicht wird, wobei für jeden der entsprechenden Zwischenzustände sowie den resultierenden Änderungszustand jeweils automatisch aus der Ausgangsgeometrie des Zahnersatzteilmodells eine zustandsspezifische Zustandsgeometrie des Zahnersatzteilmodells unter Einhaltung der geometrischen Anpassungskriterien berechnet wird,
wobei jede der benutzerdefinierten Veränderungen mittels einer grafischen Benutzeroberfläche (110) auf der Anzeigevorrichtung (108) angezeigt wird, wobei das Anzeigen der jeweiligen benutzerdefinierten Veränderung jeweils ein Anzeigen des dynamischen Durchlaufens der jeweiligen Sequenz von Zwischenzuständen bis zum Erreichen des jeweiligen Änderungszustands durch das Zahnersatzteilmodell mit den jeweiligen hierfür berechneten zustandsspezifische Zustandsgeometrien umfasst,
• Festlegen einer aus dem patientenindividuellen Anpassen des Zahnersatzteilmodells resultierenden Änderungsgeometrie als zum Herstellen des patientenindividuellen Zahnersatzteils zu verwendende patientenindividuellen Zahnersatzteilgeometrie.

20. Bearbeitungssystem (160) zum Herstellen eines patientenindividuellen Zahnersatzteils, wobei das Bearbeitungssystem ein Computersystem nach Anspruch 19 umfasst sowie eine Bearbeitungsvorrichtung (130, 150) zum Herstellen des patientenindividuellen Zahnersatzteils (140) aus Zahnersatzmaterial (138) unter Verwendung der patientenindividuellen Zahnersatzteilgeometrie.

## Claims

1. A computer-implemented method for modelling a patient-individualised denture part (140), wherein the method comprises:
• providing a digital three-dimensional patient situation model (118), wherein the patient situation model defines patient-specific delimiting surfaces (120, 121) of one or more objects of a set of the patient's teeth which define a patient situation geometry to which the denture part is to be adapted during the course of the modelling,
• providing a first digital three-dimensional denture part model (114) in a starting state, wherein the first denture part model defines denture part-specific delimiting surfaces (116) of the denture part which define a denture part geometry,
wherein the first denture part model, in the starting state, has a denture part geometry in the form of a starting geometry,
• providing one or more geometric adaptation criteria defined using the patient-specific delimiting surfaces, which criteria must be satisfied by the denture part-specific delimiting surfaces during the course of a patient-individualised adaptation of the denture part geometry to the patient situation geometry,
• adapting the denture part geometry of the first denture part model to the patient situation geometry of the patient situation model in a patient-individualised manner,
wherein the patient-individualized adaptation process comprises arranging the first denture part model in a starting position provided by the patient situation model for the denture part,
wherein the patient-individualized adaptation process also comprises repeatedly interactively making user-defined changes to the arranged first denture part model, wherein the first denture part model dynamically passes through a sequence of intermediate states during the course of each of the user-defined changes until a change state resulting from each user-defined change is reached, wherein, for each of the corresponding intermediate states and also the resulting change state, a state-specific state geometry of the first denture part model is automatically calculated from the starting geometry of the first denture part model whilst satisfying the geometric adaptation criteria,
wherein each of the user-defined changes is displayed by means of a graphical user surface (110) on a display device (108), wherein each display of a user-defined change comprises a display of the first denture part model dynamically passing through the relevant sequence of intermediate states until the corresponding change state has been reached, with the relevant state-specific state geometries calculated for this,
• using a change geometry resulting from the patient-individualised adaptation of the first denture part model to provide a patient-individualised denture part geometry for the production of the patient-individualised denture part.

2. The method according to claim 1, wherein providing the resulting change geometry comprises using the resulting change geometry as a patient-individualised denture part geometry.

3. The method according to claim 1, wherein providing the resulting change-geometry comprises transferring the resulting change geometry to a second digital three-dimensional denture part model, wherein the second denture part model has a higher resolution than the first denture part model.

4. The method according to claim 1, wherein the geometric adaptation criteria define one or more admissible maximum and/or minimum values for positive and/or negative distances between patient-specific delimiting surfaces of the patient situation model and denture part-specific delimiting surfaces of the denture part model.

5. The method according to claim 1, wherein the geometric adaptation criteria define one or more admissible minimum values for positive distances between denture part-specific delimiting surfaces of the denture part model.

6. The method according to one of the preceding claims, wherein the geometric adaptation criteria are structured hierarchically, and, in the event of incompatible geometric adaptation criteria, individual geometric adaptation criteria are given priority over one or more other geometric adaptation criteria in accordance with the hierarchical structure.

7. The method according to one of the preceding claims, wherein the user-defined changes are displayed in real time.

8. The method according to one of the preceding claims, wherein the user-defined changes each comprise at least one of the following changes defined by an interactive user input: a scaling of an extension of the denture part model in a predefined extension direction of the denture part model; a shift of the denture part model relative to the patient situation model; and a rotation of the denture part model relative to the patient situation model.

9. The method according to one of the preceding claims, wherein inputting the user-defined changes in each case comprises selecting and interactively processing at least one area of a delimiting surface of the denture part model presented visually on the graphical user surface by means of an interactive digital processing tool (122) provided by the graphical user surface.

10. The method according to claim 9, wherein the interactive processing comprises deforming an area and/or trimming a volume portion of the denture part geometry delimited by the area.

11. The method according to one of the preceding claims, wherein arranging the denture part model in the starting position comprises automatically adapting the denture part model to a preparation margin for the denture part defined in the patient situation model.

12. The method according to one of the preceding claims, wherein the method also comprises:
• choosing a change state of the first denture part model,
• simulating a chewing motion for the selected change state of the first denture part model, wherein the simulation of the chewing motion comprises calculating a sequence of relative positions of the denture part model passed through dynamically to an antagonist of the denture part model comprised by the patient situation model, wherein at least one occlusal delimiting face of the denture part model and an occlusal delimiting face of the antagonist are displayed on the display device by means of the graphical user surface for each of the relative positions.

13. The method according to claim 12, wherein, for each of the individual relative positions of the dynamic sequence, areas of the occlusal delimiting face of the denture part model which penetrate the occlusal delimiting face of the antagonist are displayed.

14. The method according to one of the preceding claims, wherein the provided denture part model in the starting state is a generic model for the denture part.

15. The method according to one of the preceding claims, wherein the objects of a set of patient's teeth comprise one or more of the following objects: a tooth, a tooth stump, gum, a denture, an implant, a periodontal apparatus, a locator, an occlusal splint, a bar, a dental prosthesis or a partial dental prosthesis, a removable partial denture, a temporary denture, a filling, or an inlay.

16. The method according to one of the preceding claims, wherein the method also comprises producing the patient-individualised denture part using the change geometry defined as patient-individualised denture part geometry.

17. The method according to one of the preceding claims, wherein the patient-specific and denture part-specific delimiting surfaces are implemented with use of one of the following methods: a polygonal mesh structure, wherein vertices of the corresponding mesh structure and/or points within the polygons of the mesh structure define the corresponding delimiting surfaces, a point cloud, wherein the points of the point cloud define the corresponding delimiting surfaces, a 3D volume data structure which comprises a voxel grid, or a 3D signed distance field.

18. A computer program product for modelling a patient-individualised denture part (140), which computer program product comprises a non-volatile, computer-readable storage medium with computer-readable program instructions for modelling the patient-individualised denture part, wherein execution of the program instructions by a processor of a computer system (100) prompts the computer system to perform a method for modelling the patient-individualised denture part, which method comprises:
• providing a digital three-dimensional patient situation model (118), wherein the patient situation model defines patient-specific delimiting surfaces (120, 121) of one or more objects of a set of the patient's teeth which define a patient situation geometry to which the denture part is to be adapted during the course of the modelling,
• providing a digital three-dimensional denture part model (114) in a starting state, wherein the denture part model defines denture part-specific delimiting surfaces (116) of the denture part which define a denture part geometry, wherein the denture part model, in the starting state, has a denture part geometry in the form of a starting geometry,
• providing one or more geometric adaptation criteria defined using the patient-specific delimiting surfaces, which criteria must be satisfied by the denture part-specific delimiting surfaces during the course of a patient-individualised adaptation of the denture part geometry to the patient situation geometry,
• adapting the denture part geometry of the denture part model to the patient situation geometry of the patient situation model in a patient-individualised manner,
wherein the patient-individualized adaptation process comprises arranging the denture part model in a starting position provided by the patient situation model for the denture part,
wherein the patient-individualized adaptation process also comprises repeatedly interactively making user-defined changes to the arranged denture part model, wherein the denture part model dynamically passes through a sequence of intermediate states during the course of each of the user-defined changes until a change state resulting from each user-defined change is reached, wherein, for each of the corresponding intermediate states and also the resulting change state, a state-specific state geometry of the denture part model is automatically calculated from the starting geometry of the denture part model whilst satisfying the geometric adaptation criteria,
wherein each of the user-defined changes is displayed by means of a graphical user surface (110) on a display device (108), wherein each display of a user-defined change comprises a display of the denture part model dynamically passing through the relevant sequence of intermediate states until the corresponding change state has been reached, with the relevant state-specific state geometries calculated for this,
• defining a change geometry, resulting from the patient-individualised adaptation of the denture part model, as patient-individualised denture part geometry to be used to produce the patient-individualised denture part.

19. A computer system (100) for modelling a patient-individualised denture part (140), wherein the computer system comprises a storage medium, a processor, an input device (104, 106) and a display device (108), wherein computer-readable program instructions for modelling the patient-individualised denture part are stored on the storage medium, wherein execution of the program instructions by the processor of the computer system prompts the computer system to perform a method for modelling the patient-individualised denture part, which method comprises:
• providing a digital three-dimensional patient situation model (118), wherein the patient situation model defines patient-specific delimiting surfaces (120, 121) of one or more objects of a set of the patient's teeth which define a patient situation geometry to which the denture part is to be adapted during the course of the modelling,
• providing a digital three-dimensional denture part model in a starting state, wherein the denture part model (114) defines denture part-specific delimiting surfaces (116) of the denture part which define a denture part geometry,
wherein the denture part model, in the starting state, has a denture part geometry in the form of a starting geometry,
• providing one or more geometric adaptation criteria defined using the patient-specific delimiting surfaces, which criteria must be satisfied by the denture part-specific delimiting surfaces during the course of a patient-individualised adaptation of the denture part geometry to the patient situation geometry,
• adapting the denture part geometry of the denture part model to the patient situation geometry of the patient situation model in a patient-individualised manner,
wherein the patient-individualized adaptation process comprises arranging the denture part model in a starting position provided by the patient situation model for the denture part,
wherein the patient-individualized adaptation process also comprises repeatedly interactively making user-defined changes to the arranged denture part model, wherein the denture part model dynamically passes through a sequence of intermediate states during the course of each of the user-defined changes until a change state resulting from each user-defined change is reached, wherein, for each of the corresponding intermediate states and also the resulting change state, a state-specific state geometry of the denture part model is automatically calculated from the starting geometry of the denture part model whilst satisfying the geometric adaptation criteria,
wherein each of the user-defined changes is displayed by means of a graphical user surface (110) on the display device (108), wherein each display of a user-defined change comprises a display of the denture part model dynamically passing through the relevant sequence of intermediate states until the corresponding change state has been reached, with the relevant state-specific state geometries calculated for this,
• defining a change geometry, resulting from the patient-individualised adaptation of the denture part model, as patient-individualised denture part geometry to be used to produce the patient-individualised denture part.

20. A processing system (160) for producing a patient-individualised denture part, wherein the processing system comprises a computer system according to claim 19 and a processing device (130, 150) for producing the patient-individualised denture part (140) from denture material (138) with use of the patient-individualised denture part geometry.

## Revendications

1. Procédé mis en œuvre par ordinateur permettant la création d'un modèle d'une prothèse dentaire (140) individualisée pour le patient, le procédé comprenant :
• la mise au point d'un modèle de situation du patient (118) tridimensionnel numérique, où le modèle de situation du patient définit des surfaces de délimitation (120, 121) spécifiques pour le patient d'un ou de plusieurs objets d'un dentier du patient, lesquels définissent une géométrie de situation du patient sur laquelle la prothèse dentaire doit être adaptée lors de la création du modèle,
• la mise au point d'un premier modèle de prothèse dentaire (114) tridimensionnel numérique dans un état de départ, où le premier modèle de prothèse dentaire définit des surfaces de délimitation (116) de la prothèse dentaire spécifiques à la prothèse dentaire, lesquelles définissent une géométrie de la prothèse dentaire,
dans lequel le premier modèle de prothèse dentaire présente dans l'état de départ une géométrie de prothèse dentaire sous la forme d'une géométrie de départ,
• la mise au point d'un ou de plusieurs critères d'adaptation géométrique définis moyennant l'emploi des surfaces de délimitation spécifiques pour le patient, lesquels sont à satisfaire par les surfaces de délimitation spécifiques à la prothèse dentaire dans le cadre d'une adaptation individualisée pour le patient de la géométrie de la prothèse dentaire à la géométrie de situation du patient,
• l'adaptation individualisée pour le patient de la géométrie de la prothèse dentaire du premier modèle de prothèse dentaire à la géométrie de situation du patient du modèle de situation de patient,
dans lequel l'adaptation individualisée pour le patient comprend un agencement du premier modèle de prothèse dentaire dans une position de départ fournie par le modèle de situation du patient pour la prothèse dentaire,
dans lequel l'adaptation individualisée pour le patient comprend en outre une exécution interactive répétée de modifications définies par l'utilisateur sur le premier modèle de prothèse dentaire agencé, où le premier modèle de prothèse dentaire, dans le cadre de chacune des modifications définies par l'utilisateur, passe respectivement de manière dynamique par une séquence d'états intermédiaires jusqu'à ce qu'un état de modification résultant de la modification définie par l'utilisateur respective soit atteint, où, pour chacun des états intermédiaires correspondants, ainsi que pour l'état de modification en résultant, une géométrie d'état spécifique à l'état du premier modèle de prothèse dentaire est calculée à partir de la géométrie de départ du premier modèle de prothèse dentaire, moyennant une satisfaction aux critères d'adaptation géométrique,
dans lequel chacune des modifications définies par l'utilisateur est indiquée sur un dispositif d'affichage (108) au moyen d'une surface d'utilisateur (110) graphique, où l'affichage de la modification définie par l'utilisateur respective comprend un affichage du déroulement dynamique de la séquence respective des états intermédiaires jusqu'à l'atteinte de l'état de modification respectif par le premier modèle de prothèse dentaire avec les géométries d'états spécifiques à l'état respectives calculées à cet effet,
• l'emploi d'une géométrie de modification résultant de l'adaptation individualisée pour le patient du premier modèle de prothèse dentaire pour la mise au point d'une géométrie de prothèse dentaire individualisée pour le patient permettant la fabrication de la prothèse dentaire individualisée pour le patient.

2. Procédé selon la revendication 1, dans lequel la mise au point de la géométrie de modification résultante comprend l'emploi d'une géométrie de modification résultante sous forme d'une géométrie de prothèse dentaire individualisée pour le patient.

3. Procédé selon la revendication 1, dans lequel la mise au point de la géométrie de modification résultante comprend un transfert de la géométrie de modification résultante sur un deuxième modèle de prothèse dentaire tridimensionnel numérique, où le deuxième modèle de prothèse dentaire présente une résolution plus grande que le premier modèle de prothèse dentaire.

4. Procédé selon la revendication 1, dans lequel I les critères d'adaptation géométriques fixent une ou plusieurs valeurs maximales et/ou minimales permissibles pour des écarts positifs et/ou négatifs entre des surfaces de délimitation spécifiques pour le patient du modèle de situation du patient et des surfaces de délimitation spécifiques à la prothèse dentaire du modèle de prothèse dentaire.

5. Procédé selon la revendication 1, dans lequel les critères d'adaptation géométrique fixent une ou plusieurs valeurs minimales permissibles pour des écarts positifs entre des surfaces de délimitation spécifiques pour le patient du modèle de prothèse dentaire.

6. Procédé selon l'une des revendications précédentes, dans lequel les critères d'adaptation géométrique sont structurés de manière hiérarchique et dans le cas de critères d'adaptation géométriques non compatibles entre eux, des critères d'adaptation géométrique individuels peuvent être accordés selon la structuration hiérarchique vis-à-vis d'un ou de plusieurs autres critères d'adaptation géométriques.

7. Procédé selon l'une des revendications précédentes, dans lequel l'affichage de la modification définie par l'utilisateur a lieu respectivement en temps réel.

8. Procédé selon l'une des revendications précédentes, dans lequel les modifications définies par l'utilisateur comprennent respectivement au moins une des modifications suivantes définies par une saisie d'utilisateur interactive : un redimensionnement d'une extension d'un modèle de prothèse dentaire le long d'une direction d'extension prédéfinie du modèle de prothèse dentaire, un décalage du modèle de prothèse dentaire par rapport au modèle de situation du patient et une rotation du modèle de prothèse dentaire par rapport au modèle de situation du patient.

9. Procédé selon l'une des revendications précédentes, dans lequel la saisie des modifications définies par l'utilisateur comprend une sélection et un façonnage interactif d'au moins une zone partielle d'une surface de délimitation du modèle de prothèse dentaire reproduit visuellement sur la surface d'utilisateur graphique au moyen d'un outil de façonnage (122) interactif numérique fourni par la surface d'utilisateur graphique.

10. Procédé selon la revendication 9, dans lequel le façonnage interactif comprend une transformation d'une zone partielle et/ou d'une découpe d'un segment volumique délimité par la zone partielle de la géométrie de prothèse dentaire.

11. Procédé selon l'une des revendications précédentes, dans lequel l'agencement du modèle de prothèse dentaire dans la position de départ comprend une adaptation automatique du modèle de prothèse dentaire à une limite de préparation définie dans le modèle de situation du patient pour la prothèse dentaire.

12. Procédé selon l'une des revendications précédentes, où le procédé comprend en outre :
• la sélection d'un état de modification du premier modèle de prothèse dentaire,
• la simulation d'un mouvement de mâchage pour l'état de modification sélectionné du premier modèle de prothèse dentaire, où la simulation du mouvement de mâchage comprend un calcul d'une séquence se déroulant de manière dynamique de positions relatives du modèle de prothèse dentaire par rapport à un antagoniste compris par le modèle de situation du patient du modèle de prothèse dentaire, où pour chacune des positions relatives, respectivement au moins une surface de délimitation occlusale du modèle de prothèse dentaire et une surface de délimitation occlusale de l'antagoniste sont affichées au moyen de la surface d'utilisateur graphique sur le dispositif d'affichage.

13. Procédé selon la revendication 12, dans lequel, pour les positions relatives individuelles de la séquence dynamique, respectivement des zones partielles de la surface de délimitation occlusale du modèle de prothèse dentaire sont indiquées, lesquelles traversent la surface de délimitation occlusale de l'antagoniste.

14. Procédé selon l'une des revendications précédentes, dans lequel, dans le cas du modèle de prothèse dentaire mis au point dans l'état de départ, il s'agit d'un modèle générique pour la prothèse dentaire.

15. Procédé selon l'une des revendications précédentes, dans lequel les objets d'un dentier de patient comprennent un ou plusieurs parmi les objets suivants : une dent, un moignon dentaire, de la gencive, une prothèse de dent, un implant, un parodonte, un localisateur, une plaque occlusale, une tige, une prothèse ou une prothèse partielle, un modèle de prothèse moulée, une prothèse dentaire provisoire, une obturation dentaire, un inlay.

16. Procédé selon l'une des revendications précédentes, où le procédé comprend en outre la fabrication d'une prothèse dentaire individualisée pour le patient moyennant l'emploi de la géométrie de modification fixée sous forme de géométrie de prothèse dentaire individualisée pour le patient.

17. Procédé selon l'une des revendications précédentes, dans lequel les surfaces de délimitations spécifiques pour le patient et spécifiques pour la prothèse dentaire sont mises en œuvre moyennant l'emploi d'un des procédés qui suit ; une structure en réseau polygonale, où des sommets de la structure en réseau correspondante et/ou des points à l'intérieur des polygones de la structure en réseau définissent les surfaces de délimitation correspondantes, un nuage de points, où les points du nuage de points définissent les surfaces de délimitation correspondantes, une structure de données de volume en 3D, laquelle comprend une grille de voxels, ou un champ de distance 3D.

18. Produit-programme informatique permettant la création d'un modèle d'une prothèse dentaire (140) individualisée pour le patient, lequel comprend un support de stockage lisible par ordinateur non volatil avec des instructions de programme lisibles par ordinateur pour créer le modèle de la prothèse dentaire individualisée pour le patient, où l'exécution des instructions de programme par un processeur d'un système informatique (100) fait en sorte que le système informatique exécute un procédé permettant la création d'un modèle d'une prothèse dentaire individualisée pour le patient, lequel comprend :
• la mise au point d'un modèle de situation du patient (118) tridimensionnel numérique, où le modèle de situation du patient définit des surfaces de délimitation (120, 121) spécifiques pour le patient d'un ou de plusieurs objets d'un dentier de patient, lesquels définissent une géométrie de situation du patient sur laquelle la prothèse dentaire doit être adaptée lors de la création du modèle,
• la mise au point d'un modèle de prothèse dentaire (114) tridimensionnel numérique dans un état de départ, où le modèle de prothèse dentaire définit des surfaces de délimitation (116) de la prothèse dentaire spécifiques à la prothèse dentaire, lesquelles définissent une géométrie de la prothèse dentaire,
où le modèle de prothèse dentaire présente dans l'état de départ une géométrie de prothèse dentaire sous la forme d'une géométrie de départ,
• la mise au point d'un ou de plusieurs critères d'adaptation géométrique définis moyennant l'emploi des surfaces de délimitation spécifiques pour le patient, lesquels sont à satisfaire par les surfaces de délimitation spécifiques à la prothèse dentaire dans le cadre d'une adaptation individualisée pour le patient de la géométrie de la prothèse dentaire à la géométrie de situation du patient,
• l'adaptation individualisée pour le patient de la géométrie de la prothèse dentaire du modèle de prothèse dentaire à la géométrie de situation du patient du modèle de situation de patient,
où l'adaptation individualisée pour le patient comprend un agencement du modèle de prothèse dentaire dans une position de départ fournie par le modèle de situation de patient pour la prothèse dentaire,
où l'adaptation individualisée pour le patient comprend en outre une exécution interactive répétée de modifications définies par l'utilisateur sur le modèle de prothèse dentaire agencé, où le modèle de prothèse dentaire, dans le cadre de chacune des modifications définies par l'utilisateur, passe respectivement de manière dynamique par une séquence d'états intermédiaires jusqu'à ce qu'un état de modification résultant de la modification définie par l'utilisateur respective soit atteint, où, pour chacun des états intermédiaires correspondants, ainsi que pour l'état de modification en résultant, une géométrie d'état spécifique à l'état du modèle de prothèse dentaire est calculée respectivement automatiquement à partir de la géométrie de départ du modèle de prothèse dentaire, moyennant la satisfaction aux critères d'adaptation géométrique,
où chacune des modifications définies par l'utilisateur est indiquée sur un dispositif d'affichage (108) au moyen d'une surface d'utilisateur (110) graphique, où l'affichage de la modification définie par l'utilisateur respective comprend un affichage du déroulement dynamique de la séquence respective des états intermédiaires jusqu'à l'atteinte de l'état de modification respectif par le modèle de prothèse dentaire avec les géométries d'états spécifiques à l'état respectives calculées à cet effet,
• la confirmation d'une géométrie de modification résultant de l'adaptation individualisée pour le patient du modèle de prothèse dentaire pour la mise au point d'une géométrie de prothèse dentaire individualisée pour le patient en tant que géométrie de prothèse dentaire individualisée pour le patient à employer pour la fabrication de la prothèse dentaire individualisée pour le patient.

19. Système informatique (100) permettant la création d'un modèle d'une prothèse dentaire (140) individualisée pour le patient, où le système informatique comprend un support de stockage, un processeur, un dispositif de saisie (104, 106) et un dispositif d'affichage (108),
dans lequel des instructions de programme lisibles par ordinateur sont stockées sur le support de stockage permettant la création d'un modèle de la prothèse dentaire individualisée pour le patient, où une exécution des instructions de programme par le processeur du système informatique fait en sorte que le système informatique exécute un procédé permettant la création d'un modèle d'une prothèse dentaire individualisée pour le patient, lequel comprend :
• la mise au point d'un modèle de situation du patient (118) tridimensionnel numérique, où le modèle de situation du patient définit des surfaces de délimitation (120, 121) spécifiques pour le patient d'un ou de plusieurs objets d'un dentier de patient, lesquels définissent une géométrie de situation du patient sur laquelle la prothèse dentaire doit être adaptée lors de la création du modèle,
• la mise au point d'un modèle de prothèse dentaire (114) tridimensionnel numérique dans un état de départ, où le modèle de prothèse dentaire définit des surfaces de délimitation (116) de la prothèse dentaire spécifiques à la prothèse dentaire, lesquelles définissent une géométrie de la prothèse dentaire,
où le modèle de prothèse dentaire présente dans l'état de départ une géométrie de prothèse dentaire sous la forme d'une géométrie de départ,
• la mise au point d'un ou de plusieurs critères d'adaptation géométrique définis moyennant l'emploi des surfaces de délimitation spécifiques pour le patient, lesquels sont à satisfaire par les surfaces de délimitation spécifiques à la prothèse dentaire dans le cadre d'une adaptation individualisée pour le patient de la géométrie de la prothèse dentaire à la géométrie de situation du patient,
• l'adaptation individualisée pour le patient de la géométrie de la prothèse dentaire du modèle de prothèse dentaire à la géométrie de situation du patient du modèle de situation de patient,
où l'adaptation individualisée pour le patient comprend un agencement du modèle de prothèse dentaire dans une position de départ fournie par le modèle de situation de patient pour la prothèse dentaire,
où l'adaptation individualisée pour le patient comprend en outre une exécution interactive répétée de modifications définies par l'utilisateur sur le modèle de prothèse dentaire agencé, où le modèle de prothèse dentaire, dans le cadre de chacune des modifications définies par l'utilisateur, passe respectivement de manière dynamique par une séquence d'états intermédiaires jusqu'à ce qu'un état de modification résultant de la modification définie par l'utilisateur respective soit atteint, où, pour chacun des états intermédiaires correspondants, ainsi que pour l'état de modification en résultant, une géométrie d'état spécifique à l'état du modèle de prothèse dentaire est calculée respectivement automatiquement à partir de la géométrie de départ du modèle de prothèse dentaire, moyennant la satisfaction aux critères d'adaptation géométrique,
où chacune des modifications définies par l'utilisateur est indiquée sur un dispositif d'affichage (108) au moyen d'une surface d'utilisateur (110) graphique, où l'affichage de la modification définie par l'utilisateur respective comprend un affichage du déroulement dynamique de la séquence respective des états intermédiaires jusqu'à l'atteinte de l'état de modification respectif par le modèle de prothèse dentaire avec les géométries d'états spécifiques à l'état respectives calculées à cet effet,
• la confirmation d'une géométrie de modification résultant de l'adaptation individualisée pour le patient du modèle de prothèse dentaire pour la mise au point d'une géométrie de prothèse dentaire individualisée pour le patient en tant que géométrie de prothèse dentaire individualisée pour le patient à employer pour la fabrication de la prothèse dentaire individualisée pour le patient.

20. Système de façonnage (160) permettant la fabrication d'une prothèse dentaire individualisée pour le patient, où le système de façonnage comprend un système informatique selon la revendication 19 ainsi qu'un dispositif de façonnage (130, 150) permettant la fabrication de la prothèse dentaire (140) individualisée pour le patient à partir de matériaux pour prothèse dentaire (138) moyennant l'emploi de la géométrie de prothèse dentaire individualisée pour le patient.
